# EUROPEAN PATENT APPLICATION

(11) **EP 3 673 883 A1**
(43) Date of publication of application: **01.07.2020**
(21) Application number: 19740884.2
(22) Date of filing: 08.01.2019
(51) Int. Cl.: A61G 5/04, G06F 3/01

(54) **TRAVEL CONTROL APPARATUS FOR ONE-PASSENGER ELECTRIC VEHICLE, TRAVEL CONTROL SYSTEM FOR ONE-PASSENGER ELECTRIC VEHICLE, AND ONE-PASSENGER ELECTRIC VEHICLE**

(30) Priority: 16.01.2018 JP 2018004581
(71) Applicant: YAMAHA HATSUDOKI KABUSHIKI KAISHA, Iwata-shi Shizuoka 438-8501 (JP)
(72) Inventor: SAITO, Tomoya, Iwata-shi, Shizuoka 438-8501 (JP); KURANUKI, Yoshiki, Iwata-shi, Shizuoka 438-8501 (JP)
(74) Representative: Stöckeler, Ferdinand
(86) International application number: PCT/JP2019/000169
(87) International publication number: WO 2019/142690

(57) **Abstract**

To control traveling of a single-seat electric vehicle by using output of a user-input detection device which has an input scheme different from those of conventionally proposed input apparatuses and which is highly versatile.

The first user-input information acquisition unit acquires first user-input information, which indicates a first user action detected by the first user-input detection device. The second user-input information acquisition unit acquires second user-input information, which indicates a second user action detected by the second user-input detection device without contact with the second part of the user's body. A multi-input controller determines a first user intention based on first user-input information acquired by a first user-input information acquisition unit. The multi-input controller determines a second user intention based on second user-input information indicative of a second user action. The multi-input controller generates a control signal for controlling traveling means based on the first user intention and the second user intention.

## Description

### TECHNICAL FIELD

The present teaching relates to a single-seat electric-vehicle travel control apparatus that controls traveling of a single-seat electric vehicle, a single-seat electric-vehicle travel control system, and a single-seat electric vehicle.

### BACKGROUND ART

As inventions related to conventional single-seat electric vehicles, for example, electric wheelchairs described in Patent Literatures 1 to 3 are known. In an electric wheelchair described in Patent Literature 1, a joystick is an input apparatus for travel control of the electric wheelchair. That is, a user operates the joystick to drive an electric motor thereby causing the electric wheelchair to travel. In an electric wheelchair described in Patent Literature 2, a CCD camera is an input apparatus for travel control of the electric wheelchair. More specifically, the CCD camera picks up an image of the face of a user. The control unit drives an electric motor thereby causing the electric wheelchair to travel based on the change in the orientation of the face of the user outputted from the CCD camera. In an electric wheelchair described in Patent Literature 3, a handrim provided on a wheel is an input apparatus for travel control of the electric wheelchair. More specifically, a user drives the electric motor thereby causing the electric wheelchair to travel by rotating the handrim. As described so far, various input apparatuses have been proposed for travel control of electric wheelchairs.

### CITATION LIST

### PATENT LITERATURE

Patent Literature 1: Japanese Patent Application Publication No. 2000-051279
Patent Literature 2: Japanese Patent Application Publication No. 11-047196
Patent Literature 3: Japanese Patent Application Publication No. 2015-013009

### SUMMARY OF INVENTION

### TECHNICAL PROBLEM

Meanwhile, for a user of a single-seat electric vehicle, different users have different body parts which are easy or hard to move. For example, among users of single-seat electric vehicles, there are users who are not able to move their arms with ease, users who are not able to move their feet with ease, and so on. Therefore, high versatility is desired for the input apparatus for travel control of a single-seat electric vehicle so that a plurality of users, who have different body parts which are difficult to move, can operate.

Accordingly, an object of the present teaching is to provide a single-seat electric-vehicle travel control apparatus that can perform travel control of a single-seat electric vehicle by using output of a user-input detection device which has an input scheme different from those of conventionally proposed input apparatuses and which is highly versatile, a single-seat electric-vehicle travel control system, and a single-seat electric vehicle.

### SOLUTION TO PROBLEM

The present inventors have conducted studies on a method for improving the versatility of the user-input detection device (input apparatus) for use in the travel control of single-seat electric vehicles. There are cases in which the user of the single-seat electric vehicle has a body part which is difficult to move. For that reason, in some cases, it is difficult for the user to perform a complex action to provide input to the user-input detection device. Therefore, it is desirable that the user-input detection device of the single-seat electric vehicle has a structure which enables detection of a simple action by the user (user action). However, as the user action is simplified, the types of user actions that the user-input detection device can detect will decrease. Such decrease in the types of user actions leads to decrease in the variety of travel control in a single-seat electric vehicle.

Then, the present inventors came up with an idea of a user-input detection device which has an input scheme different from those of conventionally proposed input apparatuses. Specifically, the present inventors considered that it will be effective to provide the single-seat electric vehicle with a plurality of user-input detection devices capable of detecting a simple user action. As a result of this, since the single-seat electric vehicle is provided with a plurality of user-input detection devices, it becomes possible to combine a plurality of user actions detected by the plurality of user-input detection devices. Thereby, in the single-seat electric vehicle, it becomes possible to detect various types of user actions by the plurality of user-input detection devices. This results in suppression of decrease in the variety of travel control in a single-seat electric vehicle.

The present inventors have also realized that since the user action is simple, the user-input detection device can detect the user action without contacting a part of the user's body. More specifically, the user-input detection device, which will not come into contact with a part of the user's body, outputs user-input information, which indicates a user action, to a controller. As such, the controller identifies the user action and determines user intention based on the user-input information. If the user action is simple, the controller can easily identify the content of the user action based on the user-input information. Thus, the present inventors have realized that if the user action is simple, the user-input detection device can detect the user action without contacting a part of the user's body.

Furthermore, the present inventors have recognized that since the user-input detection device can detect the user action without contacting a part of the user's body as described above, the user-input detection device can detect the user actions of various parts of the user's body. Therefore, the present inventors have realized that there is no need of preparing a user-input detection device, which has a structure suitable for a body part which a user can move with ease, for each user. More specifically, for a user who is able to move its arm with ease, an arm-operable joystick is suitable as the user-input detection device. For a user who is able to move its foot with ease, a foot-operable switch is suitable as the user-input detection device. In this way, in a user-input detection device which detects the user action by contacting a part of the user's body, a suitable user-input detection device differs depending on the body part which the user can move with ease. On the other hand, when the user-input detection device detects a user action without contacting a part of the user's body, for example, for a user who is able to move its arm with ease, the user-input detection device may detect the action of an arm without contacting the arm, and for a user who is able to move its foot with ease, the user-input detection device may detect the action of a foot without contacting the foot. For that reason, it is possible to use a common user-input detection device between a user who is able to move its arm with ease and a user who is able to move its foot with ease. As a result of this, it becomes not necessary to prepare different user-input detection devices for each user, thus improving the versatility of the user-input detection device.

In order to solve the above described problems, the present teaching adopts the following configuration.

A single-seat electric-vehicle travel control apparatus of (1) is a single-seat electric-vehicle travel control apparatus for performing travel control of a single-seat electric vehicle, the single-seat electric vehicle including:
a body frame;
a seat supported by the body frame, the seat being for a user to sit;
one or more drive wheels supported by the body frame;
a power supply supported by the body frame; and
traveling means including a motive power source which rotates the one or more drive wheels by receiving supply of power from the power supply, the traveling means being capable of causing the single-seat electric vehicle to move forward and backward, and to make a left turn and a right turn, wherein
a first user-input detection device is not a part of the single-seat electric-vehicle travel control apparatus, and detects a first user action of a first part of the user's body or a first user action of a first user-input representing member supported by the user's body to output first user-input information indicative of the first user action,
a second user-input detection device is not a part of the single-seat electric-vehicle travel control apparatus, and detects a second user action of a second part of the user's body or a second user action of a second user-input representing member supported by the user's body without contacting the second part of the user's body or the second user-input representing member to output second user-input information indicative of the second user action,
the second part of the user's body is a part different from the first part of the user's body, and
the second user-input representing member is a member different from the first user-input representing member, and
the single-seat electric-vehicle travel control apparatus including:
   a first user-input information acquisition unit of (A);
   a second user-input information acquisition unit of (B); and
   a multi-input controller of (C), wherein
      (A): the first user-input information acquisition unit acquires the first user-input information indicative of the first user action detected by the first user-input detection device,
      (B): the second user-input information acquisition unit acquires the second user-input information indicative of the second user action detected by the second user-input detection device without contact with the second part of the user's body or the second user-input representing member,
      (C): the multi-input controller determines a first user intention regarding travel control of the single-seat electric vehicle based on the first user-input information acquired by the first user-input information acquisition unit,
   the multi-input controller determines a second user intention regarding travel control of the single-seat electric vehicle based on the second user-input information indicative of the second user action detected by the second user-input detection device without contact with the second part of the user's body or the second user-input representing member, and
   the multi-input controller generates a control signal for controlling the traveling means based on the first user intention and the second user intention.

In the single-seat electric-vehicle travel control apparatus according to (1), a novel user-input information acquisition method is adopted. More specifically, the single-seat electric-vehicle travel control apparatus according to (1) includes a first user-input information acquisition unit and a second user-input information acquisition unit. The first user-input information acquisition unit acquires first user-input information, which indicates a first user action detected by the first user-input detection device, from the first user-input detection device. The second user-input information acquisition unit acquires second user-input information, which indicates a second action detected by the second user-input detection device without contact with the second part of the user's body or the second user-input representing member, from the second user-input detection device. In this way, in the single-seat electric-vehicle travel control apparatus according to (1), a novel user-input information acquisition method, in which two pieces of user-input information indicative of two user actions at least one of which is detected in a contactless manner are acquired, is adopted.

In the single-seat electric-vehicle travel control apparatus according to (1), even if a novel user-input information acquisition method as described above is adopted, decrease in the variety of travel control in the single-seat electric vehicle is suppressed. More specifically, the single-seat electric-vehicle travel control apparatus includes a first user-input information acquisition unit and a second user-input information acquisition unit. The first user-input information acquisition unit acquires first user-input information, which indicates a first user action detected by the first user-input detection device, from the first user-input detection device. The second user-input information acquisition unit acquires second user-input information, which indicates a second user action detected by the second user-input detection device without contact with a second part of the user's body or a second user-input representing member, from the second user-input detection device. In this way, as a result of the first user-input information and the second user-input information being acquired, the number of user actions become the number of combinations between the first user actions and the second user action. For that reason, even if the first user action of a first body part or a first user-input representing member is simple, and also the second user action of a second body part or the second user-input representing member is simple, decrease in the number of user actions will be suppressed. As a result of that, decrease in the variety of travel control in a single-seat electric vehicle will be suppressed.

Further, according to the single-seat electric-vehicle travel control apparatus according to (1), the versatility of the second user-input detection device will be improved. More specifically, as described above, the second user action of the second body part or the second user-input representing member is simple. If the second user action is simple, the multi-input controller can determine the second user intention based on the second user-input information, which indicates the second user action detected without contact with the second body part or the second user-input representing member. For that reason, the second user-input detection device may detect a second user action without contacting a second body part or a second user-input representing member.

By the second user-input detection device detecting a second user action without contacting a second body part or a second user-input representing member as described above, the second user-input detection device can detect actions of various parts of the user's body in the manner as described below. More specifically, the second user-input detection device may, for example, detect an action of an arm without contacting the arm for a user who is able to move its arm with ease. Moreover, the second user-input detection device may detect an action of a foot without contacting the foot for a user who is able to move its foot with ease. In this way, a common second user-input detection device can be used between a user who is able to move its arm with ease and a user who is able to move its foot with ease. For that reason, there is no need of preparing a second user-input detection device, which has a structure suitable for a body part which a user can move with ease, for each user. This obviates the need of preparing a different second user-input detection device for each user, thus improving the versatility of the second user-input detection device.

A single-seat electric-vehicle travel control apparatus of (2) is the single-seat electric-vehicle travel control apparatus according to (1), wherein
the one or more drive wheels include a left drive wheel which is supported by the body frame at a position further leftward than a center of the body frame in a body frame left-right direction, and a right drive wheel which is supported by the body frame at a position further rightward than the center of the body frame in the body frame left-right direction, and
the motive power source causes a difference between a rotational speed of the left drive wheel and a rotational speed of the right drive wheel when causing the single-seat electric vehicle to make a left turn or a right turn.

A single-seat electric-vehicle travel control system of (3) includes:
the first user-input detection device;
the second user-input detection device; and
the single-seat electric-vehicle travel control apparatus according to (1) or (2).

A single-seat electric-vehicle travel control system of (4) is the single-seat electric-vehicle travel control system according to (3), wherein
the second user-input detection device includes an image sensor.

A single-seat electric-vehicle travel control system of (5) is the single-seat electric-vehicle travel control system according to (3) or (4), wherein
the first user-input detection device detects the first user action of the first part of the user's body or the first user action of the first user-input representing member without contacting the first part of the user's body or the first user-input representing member.

According to the single-seat electric-vehicle travel control system of (5), the versatility of the first user-input detection device will be improved. More specifically, as described above, the first user action of the first body part or the first user-input representing member is simple. If the first user action is simple, the multi-input controller can determine the first user intention based on the first user-input information which indicates the first user action detected without contact with the first body part or the first user-input representing member. For that reason, the first user-input detection device may detect the first user action without contacting the first body part or the first user-input representing member.

By the first user-input detection device detecting a first user action without contacting a first body part or a first user-input representing member as described above, the first user-input detection device can detect first user actions of various parts of the user's body in the manner as described below. More specifically, the first user-input detection device may, for example, detect an action of an arm without contacting the arm for a user who is able to move its arm with ease. Moreover, the first user-input detection device may detect an action of a foot without contacting the foot for a user who is able to move its foot with ease. In this way, a common first user-input detection device can be used between a user who is able to move its arm with ease and a user who is able to move its foot with ease. For that reason, there is no need of preparing a first user-input detection device, which has a structure suitable for a body part which a user can move with ease, for each user. This obviates the need of preparing a different first user-input detection device for each user, thus improving the versatility of the first user-input detection device.

A single-seat electric-vehicle travel control system of (6) is the single-seat electric-vehicle travel control system according to (5), wherein
the first user-input detection device includes an image sensor.

A single-seat electric-vehicle travel control system of (7) is the single-seat electric-vehicle travel control system according to (3), wherein
the first user-input detection device detects the first user action of the first part of the user's body or the first user action of the first user-input representing member by contacting the first part of the user's body or the first user-input representing member.

A single-seat electric-vehicle travel control system of (8) is the single-seat electric-vehicle travel control system according to (7), wherein
the second user-input detection device includes an image sensor, and
the multi-input controller determines that the second user intention is stopping the single-seat electric vehicle upon acquisition of the second user-input information indicative of the second user action which implies poor physical condition of the user.

A single-seat electric-vehicle travel control system of (9) is the single-seat electric-vehicle travel control system according to (7) or (8), wherein
the first user-input detection device detects the first user action of the first part of the user's body, and
the first user-input detection device includes any one of a joystick, a handle, a lever, a button, or a handrim, with which the first part of the user's body comes into contact.

A single-seat electric-vehicle travel control system of (10) is the single-seat electric-vehicle travel control system according to any one of (3) to (9), wherein
the second user-input detection device detects at least one of the second user actions of the head, jaw, face, eyeballs, eyelids, nose, mouth, tongue, ears, shoulders, hands, arms, elbows, knees, feet or a center of gravity of the body of the user.

A single-seat electric-vehicle travel control system of (11) is the single-seat electric-vehicle travel control system according to any one of (3) to (10), wherein
the second user-input detection device detects the second user action by extracting a plurality of feature points in the second part of the user's body.

According to the single-seat electric-vehicle travel control system of (11), the multi-input controller can easily determine the second user intention. More specifically, the size of the plurality of feature points in the second body part is smaller than the size of the second body part. On the other hand, the magnitude of movement of the plurality of feature points in the second body part is the same as the magnitude of movement of the second body part. Comparing a first case in which a large second body part moves by a predetermined distance with a second case in which a small plurality of feature points move by a predetermined distance, the movement is detected more easily in the second case than in the first case. Therefore, the multi-input controller can easily identify the movement of the second body part by identifying movement of the plurality of feature points based on the second user-input information. As a result, the multi-input controller can easily determine the second user intention.

A single-seat electric vehicle of (12) includes:
the body frame;
a seat supported by the body frame, the seat being for the user to sit;
one or more drive wheels supported by the body frame;
a power supply supported by the body frame;
traveling means which includes a motive power source for rotating the one or more drive wheels by receiving supply of power from the power supply, the traveling means being capable of causing a single-seat electric vehicle to move forward and backward, and to make a left turn and a right turn; and
the single-seat electric-vehicle travel control system according to any one of claims 3 to 11.

According to the single-seat electric vehicle of (12), for the same reason as with the single-seat electric-vehicle travel control apparatus according to (1), decrease in the variety of travel control in a single-seat electric vehicle is suppressed, and the versatility of the second user-input detection device is improved.

The above described purposes and other purposes, features, aspects, and benefits of the present teaching will become further apparent from the following detailed description of embodiments of the present invention which is to be presented in association with the appended drawings.

As used herein, the term "and/or" includes any and all combinations of one or more of the associated listed items.

The terms "including," "comprising" or "having" and variations thereof, when used in this specification, specify the presence of stated features, steps, operations, elements, components, and/or their equivalents but do not preclude the presence or addition of one or more other features, steps, actions, elements, components, and/or groups thereof.

Unless otherwise defined, all terms (including technical and scientific terms) used herein have the same meaning as commonly understood by one having ordinary skill in the art to which this teaching belongs.

It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

In describing the teaching, it will be understood that a number of techniques and steps are disclosed. Each of these has individual benefit and each can also be used in conjunction with one or more, or in some cases all, of the other disclosed techniques. Accordingly, for the sake of clarity, this description will refrain from repeating every possible combination of the individual steps in an unnecessary fashion. Nevertheless, the specification and claims should be read with the understanding that such combinations are entirely within the scope of the teaching and the claims.

In the following description, for purposes of explanation, numerous specific details are set forth in order to provide a thorough understanding of the present teaching. It will be evident, however, to one skilled in the art that the present teaching may be practiced without these specific details. The present disclosure is to be considered as an exemplification of the teaching, and is not intended to limit the teaching to the specific embodiments illustrated by the figures or description below.

### ADVANTAGEOUS EFFECTS OF INVENTION

The present teaching can perform travel control of a single-seat electric vehicle by using output of a user-input detection device which has an input scheme different from those of conventionally proposed input apparatuses and which is highly versatile.

### BRIEF DESCRIPTION OF DRAWINGS

[FIG. 1] FIG. 1 is a configuration diagram of a single-seat electric vehicle 1.
[FIG. 2] FIG. 2 is a view of the single-seat electric vehicle 1 when viewed from above.
[FIG. 3] FIG. 3 is a diagram showing the relationship between actions of the left hand (left hand actions) of a user 200 and modeled left-hand image data MLID (first user-input information).
[FIG. 4] FIG. 4 is a diagram showing the relationship between actions of the face (face actions) of a user 200 and modeled face image data MFID (second user-input information).
[FIG. 5] FIG. 5 is a flowchart to show actions of a multi-input controller 102.
[FIG. 6] FIG. 6 is a configuration diagram of a single-seat electric vehicle 1a.
[FIG. 7] FIG. 7 is a flowchart to show actions of a multi-input controller 102.
[FIG. 8] FIG. 8 is a block diagram of a single-seat electric-vehicle travel control system 100b.
[FIG. 9] FIG. 9 is a view of a single-seat electric vehicle 1b when viewed from behind B, a view of the single-seat electric vehicle 1b when viewed from the left L, and a view of the single-seat electric vehicle 1b when viewed from the right R.

### DESCRIPTION OF EMBODIMENTS

### (First embodiment)

### [General configuration of single-seat electric vehicle]

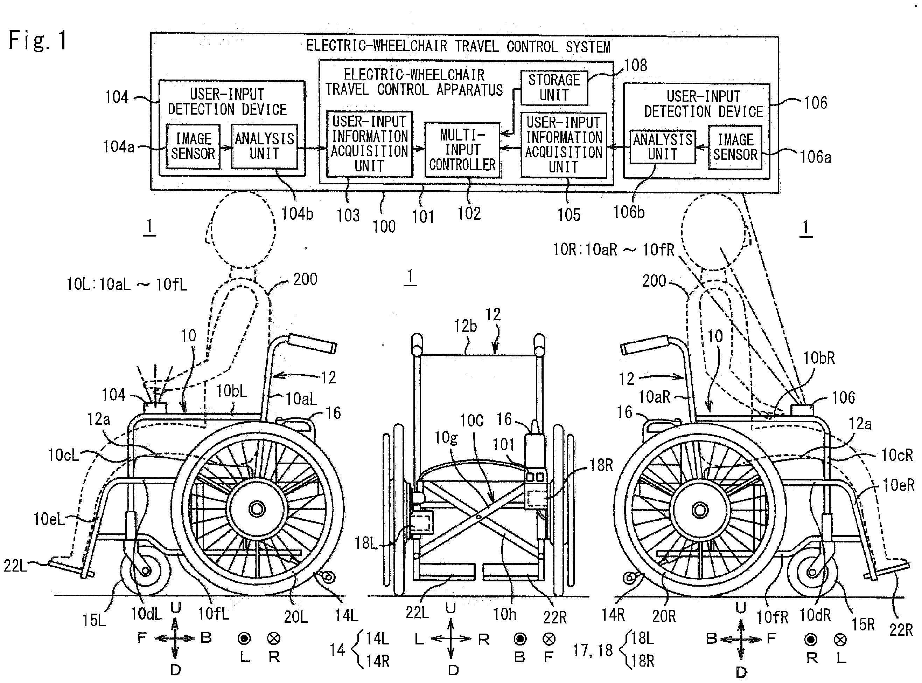

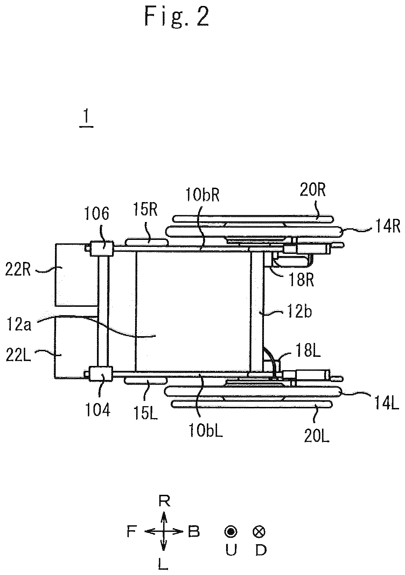

Hereinafter, a general configuration of a single-seat electric vehicle 1 will be described with reference to the drawings. FIG. 1 is a configuration diagram of a single-seat electric vehicle 1. FIG. 1 shows a view of the single-seat electric vehicle 1 when viewed from behind; a view of the single-seat electric vehicle 1 when viewed from the left; a view of the single-seat electric vehicle 1 when viewed from the right; and a block diagram of a single-seat electric-vehicle travel control system 100. FIG. 2 is a view of the single-seat electric vehicle 1 when viewed from above.

Hereinafter, the body frame 10 frontward direction is referred to as a frontward direction "F". The body frame 10 backward direction is referred to as a backward direction "B". The body frame 10 leftward direction is referred to as a leftward direction "L". The body frame 10 rightward direction is referred to as a rightward direction "R". The body frame 10 upward direction is referred to as an upward direction "U". The body frame 10 downward direction is referred to as a downward direction "D". The body frame 10 front-back direction is referred to as a front-back direction "FB". The body frame 10 left-right direction is referred to as a left-right direction "LR". The body frame 10 up-down direction is referred to as an up-down direction "UD". The body frame 10 frontward direction refers to a frontward direction with reference to a user who is seated in the single-seat electric vehicle 1. The body frame 10 backward direction refers to a backward direction with reference to a user who is seated in the single-seat electric vehicle 1. The body frame 10 leftward direction refers to a leftward direction with reference to a user who is seated in the single-seat electric vehicle 1. The body frame 10 rightward direction refers to a rightward direction with reference to a user who is seated in the single-seat electric vehicle 1. The body frame 10 upward direction refers to an upward direction with reference to a user who is seated in the single-seat electric vehicle 1. The body frame 10 downward direction refers to a downward direction with reference to a user who is seated in the single-seat electric vehicle 1.

In the present specification, an axis or a member extending in the front-back direction does not necessarily refer to only an axis or a member that is parallel with the front-back direction. An axis or a member extending in the front-back direction refers to an axis or a member that is inclined within a range of ±45° with respect to the front-back direction. Similarly, herein, an axis or a member extending in the up-down direction refers to an axis or a member that is inclined within a range of ±45° with respect to the up-down direction. Likewise, an axis or a member extending in the left-right direction refers to an axis or a member that is inclined within a range of ±45° with respect to the left-right direction.

In the present description, the phrase "a first member is supported by a second member" includes a case in which the first member is attached to the second member so as to be immovable with respect to the second member (that is, is secured thereto), and a case in which the first member is attached to the second member so as to be movable with respect to the second member. Further, the phrase "the first member is supported by the second member" includes both of a case in which the first member is directly attached to the second member and a case in which the first member is attached to the second member via a third member.

In the present specification, the phrase "the first member and the second member which are aligned in the front-back direction" shows the following state. That is a state in which when the first member and the second member are viewed in a direction perpendicular to the front-back direction, both of the first member and the second member are disposed on any straight line indicative of the front-back direction. In the present specification, the phrase "the first member and the second member which are aligned in the front-back direction when viewed in the up-down direction" shows the following state. That is, when the first member and the second member are viewed in the up-down direction, both of the first member and the second member are disposed on any straight line indicative of the front-back direction. In this case, when the first member and the second member are viewed in the left-right direction which is different from the up-down direction, either one of the first member and the second member may not be disposed on any straight line indicative of the front-back direction. Note that the first member and the second member may be in contact or overlapped with each other. The first member and the second member may also be apart from each other. A third member may be present between the first member and the second member. This definition will be applied to directions other than the front-back direction.

In the present description, the phrase "a first member is disposed further forward than a second member" refers to the following state. The first member is disposed in front of a plane which passes through a front end of the second member and is orthogonal to a front-back direction. In this case, the first member and the second member may be aligned or may not be aligned in the front-back direction. This definition will be applied to directions other than the front-back direction.

In the present description, the phrase "a first member is disposed in front of a second member" refers to the following state. At least a part of the first member is disposed in a region over which the second member passes when it is moved in parallel with a frontward direction. Therefore, the first member may fit in the region over which the second member passes when it is moved in parallel with the frontward direction, or protrude from the region over which the second member passes when it is moved in parallel with the frontward direction. In this case, the first member and the second member are aligned in the front-back direction. This definition will be applied to directions other than the front-back direction.

In the present specification, the phrase "the first member is disposed in front of the second member when viewed in the left-right direction" refers to the following state. The first member and the second member are aligned in the front-back direction when viewed in the left-right direction, and the front end of the first member is disposed further forward than the front end of the second member when viewed in the left-right direction. In this definition, the first member and the second member may not be aligned in the front-back direction in a three-dimensional space. This definition will be applied to directions other than the front-back direction.

In the present specification, unless otherwise specified, each part of the first member is defined as follows. A front part of the first member means a front half of the first member. A rear part of the first member means a rear half of the first member. A left part of the first member means a left half of the first member. A right part of the first member means a right half of the first member. An upper part of the first member means an upper half of the first member. A lower part of the first member means a lower half of the first member. An upper end of the first member means the end of the first member in the upward direction. A lower end of the first member means the end of the first member in the downward direction. A front end of the first member means the end of the first member in the frontward direction. A rear end of the first member means the end of the first member in the backward direction. A right end of the first member means the end of the first member in the rightward direction. A left end of the first member means the end of the first member in the leftward direction. An upper end part of the first member means the upper end and its vicinity of the first member. A lower end part of the first member means the lower end and its vicinity of the first member. A front end part of the first member means the front end and its vicinity of the first member. A rear end part of the first member means the rear end and its vicinity of the first member. A left end part of the first member means the left end and its vicinity of the first member. A right end part of the first member means the right end and its vicinity of the first member. The first member means a member constituting the single-seat electric vehicle 1.

The single-seat electric vehicle 1 according to the present embodiment is an electric wheelchair. As shown in FIG. 1, the single-seat electric vehicle 1 includes a body frame 10, a seat 12, one or more drive wheels 14, a left caster 15L, a right caster 15R, a power supply 16, traveling means 17, a left handrim 20L, a right handrim 20R, a left footrest 22L, a right footrest 22R, and a single-seat electric-vehicle travel control system 100. The body frame 10 is a main body of the single-seat electric vehicle 1. The body frame 10 is constituted by connecting a plurality of metal pipes. The body frame 10 includes a body frame left part 10L, a body frame right part 10R, and a body frame connecting part 10C.

The body frame left part 10L is disposed further leftward in the leftward direction L than a center of the body frame 10 in the left-right direction LR. The body frame left part 10L includes a left handle frame 10aL, a left elbow-rest frame 10bL, a left caster frame lOcL, a left seat frame 10dL, a left footrest frame 10eL, and a left under frame 10fL. The left handle frame 10aL is disposed at a rear part of the single-seat electric vehicle 1. The left handle frame 10aL extends in the up-down direction UD. The upper end part of the left handle frame 10aL is bent toward backward direction B. The upper end part of the left handle frame 10aL is used as a handle.

The left elbow-rest frame 10bL supports the left arm of a user 200. The left elbow-rest frame 10bL extends from the left handle frame 10aL in the frontward direction F. The rear end of the left elbow-rest frame 10bL is fixed to the left handle frame 10aL by welding. The left caster frame 10cL extends from the front end of the left elbow-rest frame 10bL in the downward direction D. The left elbow-rest frame 10bL and the left caster frame 10cL have a structure formed by bending a single metal pipe.

The left seat frame 10dL extends from the left handle frame 10aL in the frontward direction F below the left elbow-rest frame 10bL in the downward direction D. The rear end of the left seat frame 10dL is fixed to the left handle frame 10aL by welding. The left footrest frame 10eL linearly extends to the downward direction D and the frontward direction F from the front end of the left seat frame 10dL. The left footrest frame 10eL is disposed further forward in the frontward direction F than the left caster frame 10eL. The left seat frame 10dL and the left footrest frame 10eL have a structure formed by bending a single metal pipe.

The left under frame 10fL extends in the front-back direction FB below the left seat frame 10dL in the downward direction D. The front end of the left under frame 10fL is fixed to the left footrest frame 10eL by welding. The lower end of the left handle frame 10aL is fixed to the left under frame 10fL by welding.

The body frame right part 10R is disposed further rightward in the rightward direction R than the center of the body frame 10 in the left-right direction LR. The body frame right part 10R includes a right handle frame 10aR, a right elbow-rest frame 10bR, a right caster frame 10cR, a right seat frame 10dR, a right footrest frame 10eR and a right under frame 10fR. The right handle frame 10aR is disposed at the rear part of the single-seat electric vehicle 1. The right handle frame 10aR extends in the up-down direction UD. Further, the upper end part of the right handle frame 10aR is bent toward the backward direction B. The upper end part of the right handle frame 10aR is used as a handle.

The right elbow-rest frame 10bR supports the right arm of the user 200. The right elbow-rest frame 10bR extends in the frontward direction F from the right handle frame 10aR. The rear end of the right elbow-rest frame 10bR is fixed to the right handle frame 10aR by welding. The right caster frame 10cR extends in the downward direction D from the front end of the right elbow-rest frame 10bR. The right elbow-rest frame 10bR and the right caster frame 10cR have a structure formed by bending a single metal pipe.

The right seat frame 10dR extends from the right handle frame 10aR in the frontward direction F below the right elbow-rest frame 10bR in the downward direction D. The rear end of the right seat frame 10dR is fixed to the right handle frame 10aR by welding. The right footrest frame 10eR linearly extends in the downward direction D and the frontward direction F from the front end of the right seat frame 10dR. The right footrest frame 10eR is disposed further frontward in the frontward direction F than the right caster frame 10cR. The right seat frame 10dR and the right footrest frame 10eR have a structure formed by bending a single metal pipe.

The right under frame 10fR extends in the front-back direction FB below the right seat frame 10dR in the downward direction D. The front end of the right under frame 10fR is fixed to the right footrest frame 10eR by welding. The lower end of the right handle frame 10aR is fixed to the right under frame 10fR by welding.

A body frame connecting part 10C connects the body frame left part 10L with the body frame right part 10R. The body frame connecting part 10C includes connecting frames 10g and 10h. The connecting frame 10g is supported by the right seat frame 10dR and the left under frame 10fL near the center of the single-seat electric vehicle 1 in the front-back direction FB. Therefore, the connecting frame lOg is inclined in the rightward direction R with respect to the left handle frame 10aL. The connecting frame 10h is supported by the left seat frame 10dL and the right under frame 10fR near the center of the single-seat electric vehicle 1 in the front-back direction FB. Therefore, the connecting frame 10h is inclined in the leftward direction L with respect to the right handle frame 10aR.

The seat 12 is supported by the vehicle body frame 10. The user 200 is seated in the seat 12. More specifically, the seat 12 includes a seat surface 12a and a backrest 12b. The seat surface 12a is a flexible sheet such as cloth. The seat surface 12a is stretched between the left seat frame 10dL and the right seat frame 10dR, as shown in FIG. 2. The seat surface 12a supports the buttocks and thighs of the user 200. The backrest 12b is a flexible sheet such as cloth. The backrest 12b is stretched between the left handle frame 10aL and the right handle frame 10aR. The backrest 12b supports the back of the user 200.

One or more drive wheels 14 include a left drive wheel 14L and a right drive wheel 14R. The left drive wheel 14L is supported by the vehicle body frame 10 at a position further leftward in the leftward direction L than the center of the body frame 10 in the left-right direction LR. The left drive wheel 14L is disposed to the left of a lower part of the left handle frame 10aL, a rear part of the left seat frame 10dL, and a rear part of the left under frame 10fL in the leftward direction L. The left drive wheel 14L can rotate about an axle extending in the left-right direction LR. Hereinafter, the direction in which the left drive wheel 14L rotates when the single-seat electric vehicle 1 moves forward is referred to as a forward rotation direction. The direction in which the left drive wheel 14L rotates when the single-seat electric vehicle 1 moves backward is referred to as a reverse rotation direction.

The right drive wheel 14R is supported by the vehicle body frame 10 at a position further rightward in the rightward direction R than the center of the vehicle body frame 10 in the left-right direction LR. The right drive wheel 14R is disposed to the right of the lower part of the right handle frame 10aR, the rear part of the right seat frame 10dR, and the rear part of the right under frame 10fR in the rightward direction R. The right drive wheel 14R can rotate about an axle extending in the left-right direction LR. Hereinafter, the direction in which the right drive wheel 14R rotates when the single-seat electric vehicle 1 moves forward is referred to as a forward rotation direction. The direction in which the right drive wheel 14R rotates when the single-seat electric vehicle 1 moves backward is referred to as a reverse rotation direction.

The left caster 15L is supported by the left caster frame 10cL at the lower end of the left caster frame 10cL. Thus, when viewed from the left L, the left caster 15L is disposed in front of the left drive wheel 14L in the frontward direction F. The left caster 15L can rotate about an axle extending in the left-right direction LR. Further, the left caster 15L can rotate around the central axis of the left caster frame 10cL extending in the up-down direction UD. That is, the left caster 15L can be steered in the leftward direction L and in the rightward direction R.

The right caster 15R is supported by the right caster frame 10cR at the lower end of the right caster frame 10cR. Thus, the right caster 15R is disposed in front of the right drive wheel 14R in the frontward direction F when viewed from the right R. The right caster 15R can rotate about an axle extending in the left-right direction LR. Further, the right caster 15R can rotate around the central axis of the right caster frame 10cR extending in the up-down direction UD. That is, the right caster 15R can be steered in the leftward direction L and the rightward direction R.

The power supply 16 is supported by the vehicle body frame 10. More specifically, the power supply 16 is disposed behind the right handle frame 10aR in the backward direction B. The power supply 16 is, for example, a lithium ion battery, a nickel hydrogen battery, or the like.

The traveling means 17 is a mechanism capable of causing the single-seat electric vehicle 1 to move forward and backward, and to make a left turn and a right turn. The traveling means 17 includes a motive power source 18. The motive power source 18 rotates at least one or more drive wheels 14 by receiving supply of power from the power supply 16. In the present embodiment, the motive power source 18 rotates the left drive wheel 14L and the right drive wheel 14R by receiving supply of power from the power supply 16. The motive power source 18 rotates the left drive wheel 14L and the right drive wheel 14R in the forward rotation direction at the same rotational speed when moving the single-seat electric vehicle 1 forward. The motive power source 18 rotates the left drive wheel 14L and the right drive wheel 14R in the reverse rotation direction at the same rotational speed when moving the single-seat electric vehicle 1 backward. The motive power source 18 causes a difference between the rotational speed of the left drive wheel 14L and the rotational speed of the right drive wheel 14R when causing the single-seat electric vehicle 1 to make a left turn or a right turn. Specifically, the motive power source 18 rotates the left drive wheel 14L in the reverse rotation direction and rotates the right drive wheel 14R in the forward rotation direction when causing the electric wheelchair 1 to make a left turn with a turning radius. The motive power source 18 rotates the left drive wheel 14L and the right drive wheel 14R in the forward rotation direction such that the rotational speed of the left drive wheel 14L is smaller than the rotational speed of the right drive wheel 14R when causing the electric wheelchair 1 to make a left turn with a large turning radius. The motive power source 18 rotates the left drive wheel 14L in the forward rotation direction and rotates the right drive wheel 14R in the reverse rotation direction when causing the electric wheelchair 1 to make a right turn with a small turning radius. The motive power source 18 rotates the left drive wheel 14L and the right drive wheel 14R in the forward rotation direction such that the rotational speed of the left drive wheel 14L is larger than the rotational speed of the right drive wheel 14R when causing the electric wheelchair 1 to make a right turn with a large turning radius.

Further, the motive power source 18 also includes a left motive power source 18L and a right motive power source 18R. The left motive power source 18L is an electric motor that rotates the left drive wheel 14L via a left speed reducer. The right motive power source 18R is an electric motor that rotates the right drive wheel 14R via a right speed reducer.

The left handrim 20L is disposed to the left of the left drive wheel 14L in the leftward direction L. The left handrim 20L is a circular ring which is fixed to the left drive wheel 14L so as to be concentric with the left drive wheel 14L when viewed from the left L. Therefore, the left handrim 20L can rotate integrally with the left drive wheel 14L.

The right handrim 20R is disposed to the right of the right drive wheel 14R in the rightward direction R. The right handrim 20R is a circular ring fixed to the right drive wheel 14R so as to be concentric with the right drive wheel 14R when viewed from the right R. Therefore, the right handrim 20R can rotate integrally with the right drive wheel 14R.

The left footrest 22L is supported by the left footrest frame 10eL at the lower end part of the left footrest frame 10eL. The left footrest 22L supports the left foot of the user 200.

The right footrest 22R is supported by the right footrest frame 10eR at the lower end part of the right footrest frame 10eR. The right footrest 22L supports the right foot of the user 200.

### [Configuration of single-seat electric-vehicle travel control system]

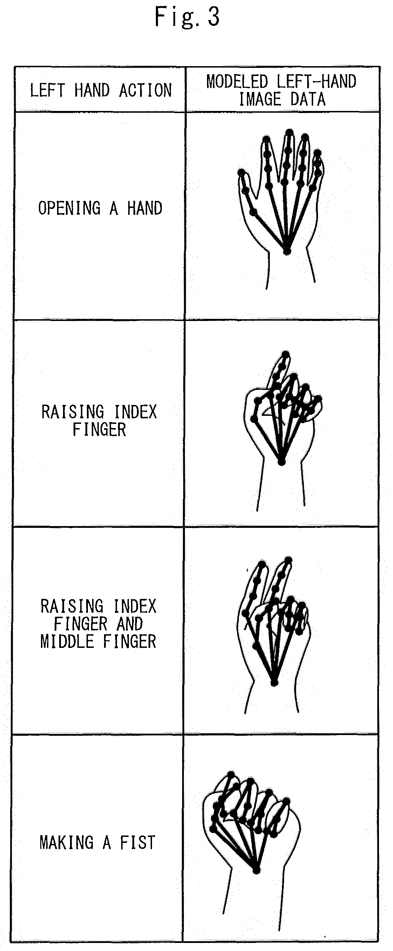

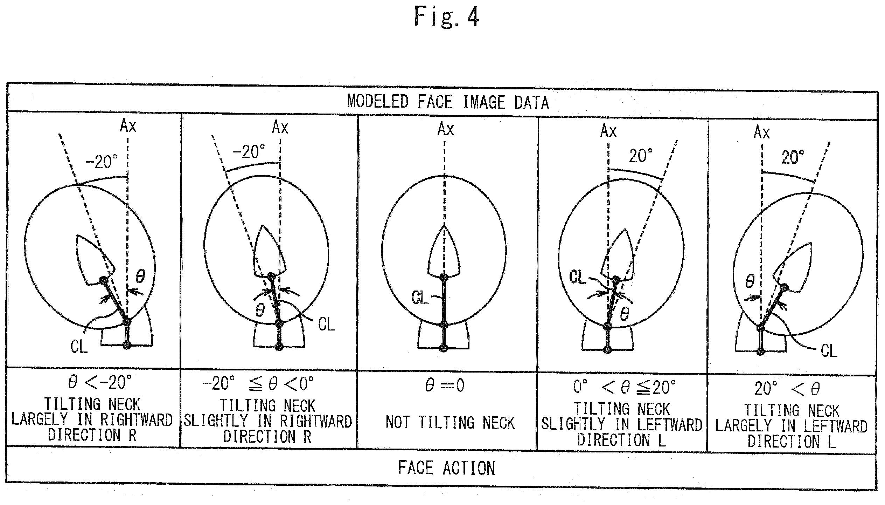

Hereinafter, the single-seat electric-vehicle travel control system 100 will be described with reference to the drawings. FIG. 3 is a diagram showing the relationship between actions of the left hand (left hand actions) of the user 200 and the modeled left-hand image data MLID (first user-input information). FIG. 4 is a diagram showing the relationship between actions of the face (face actions) of the user 200 and the modeled face image data MFID (second user-input information).

As used herein, "an action" includes an action that the user moves a part of its body, and an action that the user maintains a part of its body in a specific posture. An action that the user moves a part of its body is, for example, an action that the user tilts the neck in an upright state in the leftward direction L. On the other hand, an action that the user maintains a part of its body in a specific posture means, for example, an action that the user tilts the neck in the leftward direction L and rests at the position.

The single-seat electric-vehicle travel control system 100 performs travel control of the single-seat electric vehicle 1. As shown in FIG. 1, the single-seat electric-vehicle travel control system 100 includes a single-seat electric-vehicle travel control apparatus 101, a user-input detection device 104 (first user-input detection device), and a user-input detection device 106 (second user-input detection device).

The user-input detection device 104 is not a part of the single-seat electric-vehicle travel control apparatus 101. That is, the user-input detection device 104 is not included in the single-seat electric-vehicle travel control apparatus 101. The user-input detection device 104 detects a left hand action (first user action) of the left hand (first body part) of the user 200 without contacting the left hand of the user 200. Furthermore, the user-input detection device 104 outputs modeled left-hand image data MLID (first user-input information) indicative of a left-hand user action. More specifically, the user-input detection device 104 is fixed to the front part of the left elbow-rest frame 10bL as shown in FIG. 2. The user-input detection device 104 includes an image sensor 104a and an analysis unit 104b. The image sensor 104a picks up an image of the left hand of the user 200 by picking up an image of a region further upward in the upward direction U than the image sensor 104a. The image sensor 104a detects a left hand action by picking up an image of the left hand of the user 200. The image sensor 104a outputs the left-hand image data LID to an analysis unit 104b. The image sensor 104a is, for example, a charge coupled device (CCD) image sensor or a complementary metal-oxide-semiconductor (CMOS) image sensor. The image sensor 104a may also be an infrared camera or a line sensor.

The analysis unit 104b extracts a plurality of feature points in the left-hand image data LID, and outputs the modeled left-hand image data MLID (first user-input information) indicative of the left hand action of the plurality of feature points to the single-seat electric-vehicle travel control apparatus 101. More specifically, the analysis unit 104b extracts joints of the left hand in the left-hand image data LID as the plurality of feature points as illustrated in FIG. 3. Furthermore, the analysis unit 104b links the plurality of feature points with a line to generate modeled left-hand image data MLID. FIG. 3 shows the modeled left-hand image data MLID corresponding to four types of left hand actions of "opening a hand", "raising index finger", "raising index finger and middle finger", and "making a fist". The analysis unit 104b outputs the modeled left-hand image data MLID to the single-seat electric-vehicle travel control apparatus 101. In this occasion, the analysis unit 104b may output only a plurality of feature points as modeled left-hand image data MLID to the single-seat electric-vehicle travel control apparatus 101, or output the plurality of feature points and the left-hand image data LID as modeled left-handed image data MLID to the single-seat electric-vehicle travel control apparatus 101. The analysis unit 104b is, for example, a microcomputer made up of a combination of a circuit board, electronic components, and an IC (integrated circuit).

The user-input detection device 106 is not a part of the single-seat electric-vehicle travel control apparatus 101. That is, the user-input detection device 106 is not included in the single-seat electric-vehicle travel control apparatus 101. The user-input detection device 106 detects face actions (second user action) of the face (second body part) of the user 200 without contacting the face of the user 200. Furthermore, the user-input detection device 106 outputs modeled face image data MFID (second user-input information) indicative of face user input. More specifically, the user-input detection device 106 is fixed to the front part of the right elbow-rest frame 10bR as shown in FIG. 2. The user-input detection device 106 includes an image sensor 106a and an analysis unit 106b. The image sensor 106a picks up an image of the face of the user 200 by picking up an image of a region further upward in the upward direction U and further backward in the backward direction B than the image sensor 106a. The image sensor 106a detects face actions by picking up an image of the face of the user 200. The image sensor 106a outputs the face image data FID to the analysis unit 106b. The image sensor 106a is, for example, a CCD image sensor or a CMOS image sensor. The image sensor 106a may be an infrared camera or a line sensor.

The analysis unit 106b extracts a plurality of feature points in the face image data FID, and outputs the modeled face image data MFID (second user-input information), which indicates the face action of the plurality of feature points, to the single-seat electric-vehicle travel control apparatus 101. More specifically, as shown in FIG. 4, the analysis unit 106b extracts the lower end of the neck, the chin, and the nose of the face in the face image data FID as the plurality of feature points. Furthermore, the analysis unit 106b generates modeled face image data MFID by linking the plurality of feature points with a line. FIG. 4 shows the modeled face image data FLID corresponding to five types of face actions of "tilting the neck largely in the leftward direction L", "tilting the neck slightly in the leftward direction L", "not tilting the neck", "tilting the neck slightly in the rightward direction R" and "tilting the neck largely in the rightward direction R". The analysis unit 106b outputs the modeled face image data MFID to the single-seat electric-vehicle travel control apparatus 101. In this occasion, the analysis unit 106b may output only the plurality of feature points as the modeled face image data MFID to the single-seat electric-vehicle travel control apparatus 101, or may output the plurality of feature points and the face image data FID as the modeled face image data MFID to the single-seat electric-vehicle travel control apparatus 101. The analysis unit 106b is, for example, a microcomputer made up of a combination of a circuit board, electronic components, and an IC (integrated circuit).

The user-input detection devices 104 and 106 as described above are realized by, for example, Leap Motion (registered trademark) of Leap Motion, Inc.

The single-seat electric-vehicle travel control apparatus 101 includes a multi-input controller 102, user-input information acquisition units 103 and 105, and a storage unit 108. The user-input information acquisition unit 103 (first user-input information acquisition unit) acquires modeled left-hand image data MLID indicative of a left-hand user action detected by the user-input detection device 104. In the present embodiment, the user-input information acquisition unit 103 acquires the modeled left-hand image data MLID outputted by the analysis unit 104b. The user-input information acquisition unit 105 (second user-input information acquisition unit) acquires modeled face image data FLID indicative of face actions detected by the user-input detection device 106. In the present embodiment, the user-input information acquisition unit 105 acquires the modeled face image data FLID outputted by the analysis unit 106b.

The multi-input controller 102 determines a first user intention regarding the travel control of the single-seat electric vehicle 1 based on the modeled left-hand image data MLID acquired by the user-input information acquisition unit 103. The first user intention is the intention of the user 200 regarding the traveling of the single-seat electric vehicle 1. The first user intention is, for example, an intention of causing the single-seat electric vehicle 1 to move forward, an intention of causing the single-seat electric vehicle to move backward, and the like.

More specifically, the multi-input controller 102 determines the left hand action of the modeled left-hand image data MLID by performing image analysis on the modeled left-hand image data MLID. That is, the multi-input controller 102 determines which one of "opening a hand", "raising index finger", "raising index finger and middle finger", and "making a fist" shown in FIG. 3 is the left hand action of the modeled left-hand image data MLID. Furthermore, the multi-input controller 102 determines the first user intention regarding the travel control of the single-seat electric vehicle 1 based on the left hand action. Therefore, the storage unit 108 stores the first user-intention table shown in Table 1. The storage unit 108 is, for example, a non-volatile memory.

### [Table 1]

**TABLE 1**

| Left hand action | Opening a hand | Raising index finger | Raising index finger and middle finger | Making a fist |
|---|---|---|---|---|
| First user intention | Stopping | Moving forward at V1 | Moving forward at V2 | Moving backward |

In the first user-intention table shown in Table 1, the left hand action and the first user intention are recorded in association with each other. The left hand action of "opening a hand" means that the first user intention is "stopping the single-seat electric vehicle 1". The left hand action of "raising index finger" means that the first user intention is "causing the single-seat electric vehicle 1 to move forward at a speed VI". The left hand action of "raising the index finger and middle finger" means that the first user intention is "causing the single-seat electric vehicle 1 to move forward at a speed V2". The left hand action of "making a fist" means that the first user intention is "causing the single-seat electric vehicle 1 to move backward".

The multi-input controller 102 determines the first user intention corresponding to the left hand action indicated by the modeled left-hand image data MLID by referring to Table 1. In the present embodiment, the multi-input controller 102 determines which one of "stopping the single-seat electric vehicle 1", "causing the single-seat electric vehicle 1 to move forward at a speed V1", "stopping the single-seat electric vehicle 1", and "causing the single-seat electric vehicle 1 to move backward" is the first user intention.

Moreover, the multi-input controller 102 determines second user intention regarding the travel control of the single-seat electric vehicle 1 based on the modeled face image data FLID which indicates the face action detected by the user-input detection device 106 without contacting the face of the user 200. The second user intention is the intention of the user 200 regarding the traveling of the single-seat electric vehicle 1. The second user intention is, for example, causing the single-seat electric vehicle 1 to make a left turn, or causing the single-seat electric vehicle 1 to make a right turn, and the like.

More specifically, the multi-input controller 102 determines the face action indicated by the modeled face image data FLID by performing image analysis on the modeled face image data FLID. That is, the multi-input controller 102 determines which one of "tilting the neck largely in the leftward direction L", "tilting the neck slightly in the leftward direction L", "not tilting the neck", "tilting the neck slightly in the rightward direction R", or "tilting the neck largely in the rightward direction R" of FIG. 4 is the face action indicated by the modeled face image data FLID based on the inclination angle of the face.

First, as shown in FIG. 4, a straight line which passes through the center (front end of the nose) of the face in an upright state and extends in the up-down direction UD when viewed from front F is defined as a vertical axis Ax. Moreover, a line linking the feature point of the jaw and the feature point of the nose is defined as a center line CL of the face. An angle formed by the center line CL and the vertical axis Ax is defined as an inclination angle θ. The inclination angle θ takes a positive value when the center line CL rotates clockwise when viewed from front F. Therefore, the inclination angle θ takes a positive value when the user tilts the neck in the leftward direction L. The inclination angle θ takes a negative value when the center line CL rotates counterclockwise when viewed from front F. Therefore, the inclination angle θ takes a negative value when the user tilts the neck in the rightward direction R.

If the inclination angle θ is more than 20°, the multi-input controller 102 determines that the face action is "tilting the neck largely in the leftward direction L". If the inclination angle θ is more than 0° and not more than 20°, the multi-input controller 102 determines that the face action is "tilting the neck slightly in the leftward direction L". If the inclination angle θ is 0°, the multi-input controller 102 determines that the face action is "not tilting the neck". If the inclination angle θ is not less than -20° and less than 0°, the multi-input controller 102 determines that the face action is "tilting the face slightly in the rightward direction R". If the inclination angle θ is less than -20°, the multi-input controller 102 determines that the face action is "tilting the face largely in the rightward direction R".

The multi-input controller 102 determines a second user intention regarding travel control of the single-seat electric vehicle 1 based on the face action. Accordingly, the storage unit 108 stores a second user-intention table shown in Table 2.

### [Table 2]

**TABLE 2**

| Face action | Tilting the neck largely in rightward direction R θ < -20° | Tilting the neck slightly in rightward direction R -20° ≤ θ < 0° | Not tilting the neck θ = 0° | Tilting the neck slightly in leftward direction L 0° < θ ≤ 20° | Tilting the neck largely in leftward direction L 20° < θ |
|---|---|---|---|---|---|
| Second user intention | Making a right turn with a small radius | Making a right turn with a large radius | No turning | Making a left turn with a large radius | Making a left turn with a small radius |

In the second user-intention table shown in Table 2, the face action and the second user intention are recorded in association with each other. The face action of "tilting the neck largely in the leftward direction L" means that the second user intention is "causing the single-seat electric vehicle 1 to make a left turn with a small radius". The face action of "tilting the neck slightly in the leftward direction L" means that the second user intention is "causing the single-seat electric vehicle 1 to make a left turn with a large radius". The face action of "not tilting the neck" means that the second user intention is "causing the single-seat electric vehicle 1 to make neither a left turn nor a right turn". The face action of "tilting the neck slightly in the rightward direction R" means that the second user intention is "causing the single-seat electric vehicle 1 to make a right turn with a large radius". The face action of "tilting the neck largely in the rightward direction R" means that the second user intention is "causing the single-seat electric vehicle 1 to make a right turn with a small radius".

The multi-input controller 102 determines the second user intention corresponding to the face action indicated by the modeled face image data FLID by referring to Table 2. The multi-input controller 102 determines which one of "causing the single-seat electric vehicle 1 to make a left turn with a small radius", "causing the single-seat electric vehicle 1 to make a left turn with a large radius", "causing the single-seat electric vehicle 1 to make neither a left turn nor a right turn", "causing the single-seat electric vehicle 1 to make a right turn with a large radius" or "causing the single-seat electric vehicle 1 to make a right turn with a small radius" is the second user intention.

The multi-input controller 102 generates a control signal to control the traveling means 17 based on the first user intention and the second user intention. In the present embodiment, the multi-input controller 102 generates a control signal for controlling the motive power source 18 based on the first user intention and the second user intention. Specifically, the multi-input controller 102 generates a control signal for performing travel control which combines the first user intention of Table 1 and the second user intention of Table 2. Table 3 is a travel control table showing travel control which combines the first user intention of Table 1 and the second user intention of Table 2. Although not shown, detailed control of the left motive power source 18L and the right motive power source 18R in each travel control is also recorded in the travel control table of Table 3. For example, when the travel control is "making a right turn on the spot", the detailed control will be "rotating the left motive power source 18L at a revolution R1 in a reverse rotation direction, and rotating the right motive power source 18R at a revolution R1 in a forward rotation direction". The storage unit 108 stores a travel control table shown in Table 3. The multi-input controller 102 determines travel control corresponding to the first user intention and the second user intention based on the travel control table of Table 3. Furthermore, the multi-input controller 102 generates a control signal for controlling the motive power source 18 based on the travel control. The motive power source 18 operates in accordance with the control signal. As a result of this, the single-seat electric vehicle 1 can make traveling of moving forward, moving backward, turning left, and turning right.

### [Table 3]

**TABLE 3**

| | | Second user intention | | | | |
|---|---|---|---|---|---|---|
| | | Making a small right turn | Making a large right turn | Not making a turn | Making a large left turn | Making a small left turn |
| First user intention | Stopping | Stopping | Stopping | Stopping | Stopping | Stopping |
| | Moving forward at V1 | Making a right turn on the spot | Making a right turn with a large radius while moving forward at V1 | Moving forward at V1 | Making a left turn with a large radius while moving forward at VI | Making a left turn on the spot |
| | Moving forward at V2 | Making a right turn on the spot | Making a right turn with a large radius while moving forward at V2 | Moving forward at V2 | Making a left turn with a large radius while moving forward at V2 | Making a left turn on the spot |
| | Moving backward | Making a right turn on the spot | Making a right turn with a large radius while moving backward | Moving backward | Making a left turn with a large radius while moving backward | Making a left turn on the spot |

The multi-input controller 102 as described above is, for example, a microcomputer which is constituted by a combination of a circuit board, electronic components, and an IC (integrated circuit). The user-input information acquisition unit 103 is, for example, a volatile memory or a non-volatile memory that temporarily stores the modeled left-hand image data MLID acquired from the user-input detection device 104.

The user-input information acquisition unit 105 is, for example, a volatile memory or a non-volatile memory that temporarily stores the modeled face image data FLID acquired from the user-input detection device 104. The volatile memory or non-volatile memory is a part of the microcomputer. Moreover, the multi-input controller 102 is an arithmetic processing unit of the microcomputer.

Note that the user-input information acquisition unit 103 may have a function of acquiring the modeled left-hand image data MLID indicative of the left hand action detected by the user-input detection device 104. Further, the user-input information acquisition unit 105 may have a function of acquiring the modeled face image data FLID indicative of the face action detected by the user-input detection device 106. Therefore, the user-input information acquisition units 103 and 105 will not be limited to the volatile memory or the non-volatile memory as long as they have the above-described functions. For example, the user-input information acquisition units 103 and 105 may be terminals of the single-seat electric-vehicle travel control apparatus 101 to which the modeled left-hand image data MLID or the modeled face image data FLID is inputted.

### [Action of single-seat electric vehicle travel control apparatus]

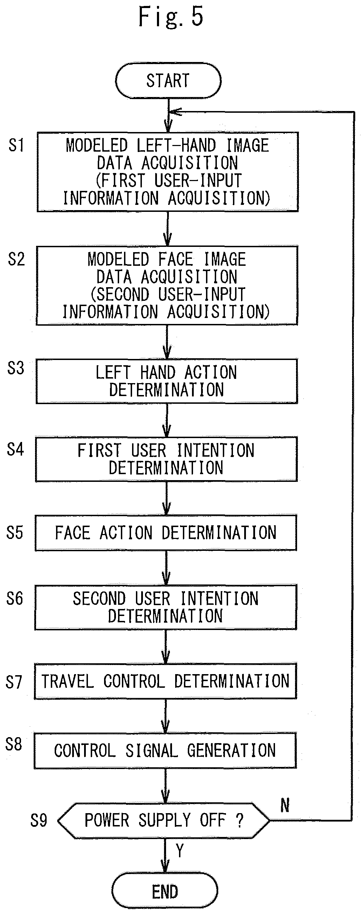

Next, the action of the single-seat electric-vehicle travel control apparatus 101 will be described with reference to the drawings. FIG. 5 is a flowchart showing actions of the single-seat electric-vehicle travel control apparatus 101. The single-seat electric-vehicle travel control apparatus 101 executes the flowchart of FIG. 5 according to the program stored by the storage unit 108.

The present control is started by switching the power supply of the single-seat electric vehicle 1 from OFF to ON. The image sensor 104a picks up an image of the left hand of the user 200, and outputs left-hand image data LID to the analysis unit 104b. The analysis unit 104b generates the modeled left-hand image data MLID based on the left-hand image data LID, and outputs the modeled left-hand image data MLID to the user-input information acquisition unit 103. The user-input information acquisition unit 103 acquires the modeled left-hand image data MLID (step S1). The user-input information acquisition unit 103 outputs the modeled left-hand image data MLID to the multi-input controller 102. As a result, the multi-input controller 102 acquires the modeled left-hand image data MLID.

Moreover, the image sensor 106a picks up an image of the face of the user 200, and outputs face image data FID to the analysis unit 106b. The analysis unit 106b generates modeled face image data MFID based on the face image data FID, and outputs the modeled face image data MFID to the user-input information acquisition unit 105. The user-input information acquisition unit 105 acquires the modeled face image data MFID (step S2). The user-input information acquisition unit 103 outputs the modeled face image data FLID to the multi-input controller 102. As a result, the multi-input controller 102 acquires the modeled face image data FLID.

Next, the multi-input controller 102 determines which one of "opening a hand", "raising index finger", "raising index finger and middle finger", and "making a fist" shown in FIG. 3 is the left hand action indicated by the modeled left-hand image data MLID (step S3). Further, based on the first user-intention table of Table 1, the multi-input controller 102 determines which one of "causing the single-seat electric vehicle 1 to stop", "causing the single-seat electric vehicle 1 to move forward at a speed V1", "causing the single-seat electric vehicle 1 to move forward at a speed V2", or "causing single-seat electric vehicle 1 to move backward" is the first user intention, which corresponds to the left hand action determined in step S3 (step S4).

Next, the multi-input controller 102 determines which one of "tilting the neck largely in the leftward direction L", "tilting the neck slightly in the leftward direction L", "not tilting the neck", "tilting the neck slightly in the rightward direction R" or "tilting the neck largely in the rightward direction R" is the face action indicated by the modeled face image data MFID (step S5). Further, based on the second user-intention table of Table 2, the multi-input controller 102 determines which one of "causing the single-seat electric vehicle 1 to make a left turn with a small radius", "causing the single-seat electric vehicle 1 to make a left turn with a large radius", "causing the single-seat electric vehicle 1 to make neither a left turn nor a right turn", "causing the single-seat electric vehicle 1 to make a right turn with a large radius", or "causing the single-seat electric vehicle 1 to make a right turn with a small radius" is the second user intention corresponding to the face action determined in step S5 (step S6).

Next, based on the travel control table of Table 3, the multi-input controller 102 determines travel control corresponding to the first user intention and the second user intention determined in steps S4 and S6 (step S7). The multi-input controller 102 generates a control signal for controlling the motive power source 18 based on the travel control determined in step S7 (step S8). The motive power source 18 operates in accordance with the control signal.

Finally, the multi-input controller 102 determines whether or not the power supply of the single-seat electric vehicle 1 has been switched from ON to OFF (step S9). When the power supply of the single-seat electric vehicle 1 has been switched from ON to OFF, the present process ends. When the power supply of the single-seat electric vehicle 1 has not been switched from ON to OFF, the present process returns to step S1.

### [Effects]

In the single-seat electric-vehicle travel control apparatus 101, a novel user-input information acquisition method is adopted. More specifically, the single-seat electric-vehicle travel control apparatus 101 includes user-input information acquisition units 103 and 105. The user-input information acquisition unit 103 acquires the modeled left-hand image data MLID, which indicates the left hand action detected by the user-input detection device 104, from the user-input detection device 104. The user-input information acquisition unit 105 acquires the modeled face image data FLID, which indicates the face action detected by the user-input detection device 106 without contacting the face of the user 200, from the user-input detection device 106. In this way, the single-seat electric-vehicle travel control apparatus 101 adopts a novel user-input information acquisition method in which two types of data: the modeled left-hand image data MLID and the modeled face image data FLID, which indicate two types of actions of the left hand action and the face action, at least one of which is detected in a contactless manner, are acquired.

In the single-seat electric-vehicle travel control apparatus 101, even if the new user-input information acquisition method as described above is adopted, a decrease in the diversity of travel control in the single-seat electric vehicle 1 will be suppressed. More specifically, the single-seat electric-vehicle travel control apparatus 101 includes user-input information acquisition units 103 and 105. The user-input information acquisition unit 103 acquires the modeled left-hand image data MLID, which indicates the left hand action detected by the user-input detection device 104, from the user-input detection device 104. The user-input information acquisition unit 105 acquires the modeled face image data FLID, which indicates a face action detected by the user-input detection device 106 without contacting the face of the user 200, from the user-input detection device 106. In this way, as a result of the modeled left-hand image data MLID and the modeled face image data FLID being acquired, the number of user actions becomes the number of combinations of the left hand actions and the face actions. In the present embodiment, since there are four types of left hand actions and five types of face actions, 20 types of user actions can be obtained. Therefore, even if the left hand action is simple and the face action is also simple, decrease in the number of user actions will be suppressed. As a result, it is possible to suppress decrease in the variety of travel control in a single-seat electric vehicle.

Further, according to the single-seat electric-vehicle travel control apparatus 101, the versatility of the user-input detection device 104 is improved. More specifically, as described above, the face action is simple. If the face action is simple, the multi-input controller can determine the second user intention based on the modeled face image data FLID (second user-input information), which indicates the face action detected without contact with the face of the user 200. Therefore, the user-input detection device 106 may detect face actions of the second user input without contacting the face of the user 200.

As a result of the user-input detection device 106 detecting face actions without contacting the face of the user 200 as described above, the user-input detection device 106 can detect actions of various parts of the body of the user 200 in the manner as described below. More specifically, the user-input detection device 106 may detect, for example, actions of an arm without contacting the arm for a user 200 who is able to move its arm with ease. Also, the user-input detection device 106 may detect actions of a foot without contacting the foot for a user 200 who is able to move its foot with ease. In this way, a common user-input detection device 106 can be used for a user 200 who is able to move its arm with ease and a user 200 who is able to move its foot with ease. For this reason, there is no need of preparing, for each user 200, a user-input detection device 106 having a structure suitable for a body part which the user can move with ease. As a result of this, it becomes not necessary to prepare a different user-input detection device 106 for each user 200, thus improving versatility of the user-input detection device 106. For the same reason as with the user-input detection device 106, the versatility of the user-input detection device 104 will be improved as well.

Further, according to the single-seat electric-vehicle travel control system 100, the multi-input controller 102 can easily determine the second user intention. More specifically, the size of the plurality of feature points in the face is smaller than the size of the face. On the other hand, the magnitude of movement of the plurality of feature points in the face is the same as the magnitude of movement of the face. Comparing a first case in which a large face moves by a predetermined distance, and a second case in which a plurality of feature points of a small size move by the predetermined distance, the movement is detected more easily in the second case than in the first case. Therefore, the multi-input controller 102 can easily identify the movement of the face by identifying the movement of the plurality of feature points based on the modeled face image data FLID. As a result of that, the multi-input controller can easily determine the second user intention.

Moreover, the multi-input controller 102 can easily determine the first user intention for the same reason as with the second user intention.

### (Second embodiment)

### [General configuration of single-seat electric vehicle]

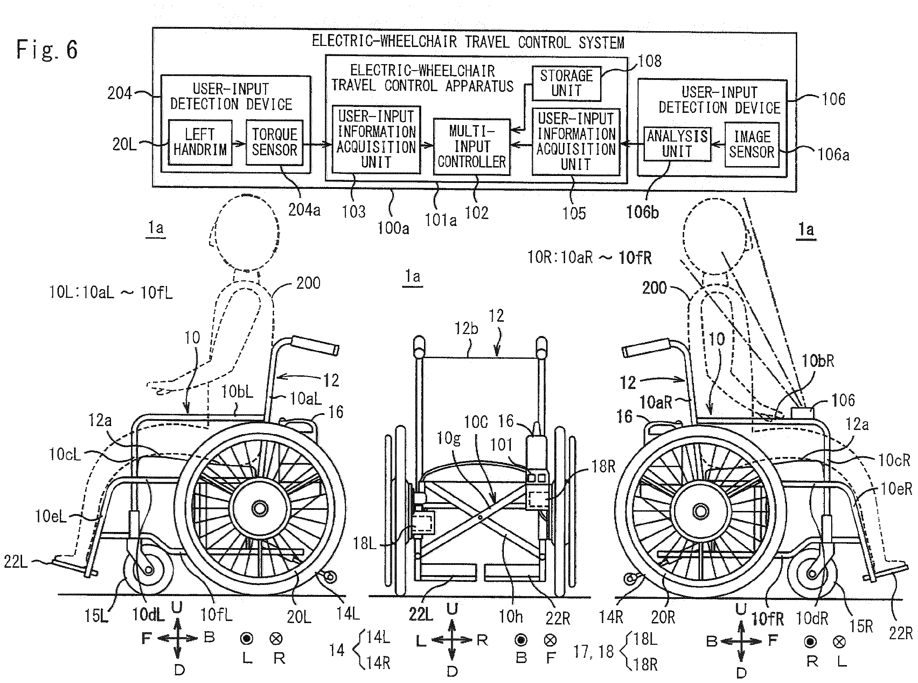

Hereinafter, a general configuration of a single-seat electric vehicle 1a will be described with reference to the drawings. FIG. 6 is a configuration diagram of the single-seat electric vehicle 1a. FIG. 6 shows a view of the single-seat electric vehicle 1a viewed from behind B, a view of the single-seat electric vehicle 1a viewed from the left L, a view of the single-seat electric vehicle 1a viewed from the right R, and a block diagram of a single-seat electric-vehicle travel control system 100a.

The single-seat electric vehicle 1a according to the present embodiment is an electric wheelchair. The single-seat electric vehicle 1a differs from the single-seat electric vehicle 1 in that the single-seat electric vehicle 1a includes a user-input detection device 204 instead of the user-input detection device 104. Hereinafter, the single-seat electric vehicle 1a will be described focusing on such differences.

The user-input detection device 204 detects left hand actions of the user 200 caused by the left hand contacting the user-input detection device 204. More specifically, the user-input detection device 204 includes a left handrim 20L and a torque sensor 204a. The left handrim 20L is disposed to the left of the left drive wheel 14L in the leftward direction L. The left handrim 20L is a circular ring fixed to the left drive wheel 14L so as to be concentric with the left drive wheel 14L when viewed from the left L. Therefore, the left handrim 20L can rotate integrally with the left drive wheel 14L. Upon the user 200 rotating the left handrim 20L in the forward rotation direction or reverse rotation direction with the left hand, the user-input detection device 204 detects torque value information Tr (to be described later) indicative of a left hand action.

The torque sensor 204a is provided on an axle of the left drive wheel 14L. As the user 200 rotates the left handrim 20L, torque is generated on the axle of the left drive wheel 14L. The torque sensor 204a detects the value (torque value) of the torque generated on the axle of the left drive wheel 14L. Then, the torque sensor 204a outputs torque value information Tr (first user-input information), which indicates a torque value, to the single-seat electric-vehicle travel control apparatus 101a. The torque sensor 204a detects a positive torque value when the left handrim 20L is rotated by the user 200 in the forward rotation direction. That is, when the torque sensor 204a detects a positive torque, the left hand action is "rotating the left handrim 20L in the forward rotation direction". The torque sensor 204a detects a torque value of 0 when the left handrim 20L is not rotated by the user 200 in the forward rotation direction or reverse rotation direction. That is, when the torque sensor 204a detects a torque value of 0, the left hand action is "not rotating the left handrim 20L". The torque sensor 204a detects a negative torque value when the left handrim 20L is rotated by the user 200 in the reverse rotation direction. That is, when the torque sensor 204a detects a negative torque value, the left hand action is "rotating the left handrim 20L in the reverse rotation direction".

The multi-input controller 102 determines the first user intention regarding the travel control of the single-seat electric vehicle 1a based on the torque value information Tr detected by the user-input detection device 204. Accordingly, the storage unit 108 stores the first user-intention table shown in Table 4.

### [Table 4]

**TABLE 4**

| | During moving forward | | | During stopping | | | During moving backward | | |
|---|---|---|---|---|---|---|---|---|---|
| Left hand action | Rotating in forward rotation direction Tr>0 | Not rotating Tr=0 | Rotating in reverse rotation direction Tr<0 | Rotating in forward rotation direction Tr>0 | Not rotating Tr=0 | Rotating in reverse rotation direction Tr<0 | Rotating in forward rotation direction Tr>0 | Not rotating Tr=0 | Rotating in reverse rotation direction Tr<0 |
| First user intention | Moving forward & acceleration | Maintaining speed | Moving forward & deceleration | Start moving forward | Stopping | Start moving backward | Moving backward & deceleration | Maintaining speed | Moving backward & acceleration |

In the first user-intention table shown in Table 4, the left hand action and the first user intention are recorded in association with each other. The left hand action "rotating left handrim 20L in the forward rotation direction" means that the user intention is "accelerating forward movement of the single-seat electric vehicle 1a" when the single-seat electric vehicle 1a is moving forward. The left hand action of "not rotating the left handrim 20L" means that the user intention is "maintaining the speed of forward movement of the single-seat electric vehicle 1a" when the single-seat electric vehicle 1a is moving forward. The left hand action of "rotating the left handrim 20L in the reverse rotation direction" means that the user intention is "decelerating forward movement of the single-seat electric vehicle 1a" when the single-seat electric vehicle 1a is moving forward. The left hand action of "rotating the left handrim 20L in the forward rotation direction" means that the user intention is "starting forward movement of the single-seat electric vehicle 1a" when the single-seat electric vehicle 1a is stopped. The left hand action of "not rotating the left handrim 20L" means that the user intention is "stopping the single-seat electric vehicle 1a" when the single-seat electric vehicle 1a is stopped. The left hand action of "rotating the left handrim 20L in the reverse rotation direction" means that the user intention is "starting backward movement of the single-seat electric vehicle 1a" when the single-seat electric vehicle 1a is stopped. The left hand action of "rotating the left handrim 20L in the forward rotation direction" means that the user intention is "decelerating backward movement of the single-seat electric vehicle 1a" when single-seat electric vehicle 1a is moving backward. The left hand action of "not rotating the left handrim 20L" means that the user intention is "maintaining the speed of backward movement of the single-seat electric vehicle 1a" when the single-seat electric vehicle 1a is moving backward. The left hand action of "rotating the left handrim 20L in the reverse rotation direction" means that the user intention is "accelerating backward movement of the single-seat electric vehicle 1a" when the single-seat electric vehicle 1a is moving backward.

The multi-input controller 102 determines a first user intention corresponding to a left hand action by referring to Table 4. The multi-input controller 102 determines which one of "accelerating forward movement of the single-seat electric vehicle 1a", "maintaining the speed of forward movement of the single-seat electric vehicle 1a", "decelerating forward movement of the single-seat electric vehicle 1a", "starting forward movement of the single-seat electric vehicle 1a", "stopping the single-seat electric vehicle 1a", "starting backward movement of the single-seat electric vehicle 1a", "decelerating backward movement of the single-seat electric vehicle 1a", "maintaining the speed of backward movement of the single-seat electric vehicle 1a", or "accelerating backward movement of the single-seat electric vehicle 1a" is the first user intention.

The configurations other than the user-input detection device 204 of the single-seat electric vehicle 1a is the same as that of the single-seat electric vehicle 1, and therefore the description thereof will be omitted.

The multi-input controller 102 generates a control signal for controlling the traveling means 17 based on the first user intention and the second user intention. In the present embodiment, the multi-input controller 102 generates a control signal for controlling the motive power source 18 based on the first user intention and the second user intention. Specifically, the multi-input controller 102 generates a control signal for performing travel control, which combines the first user intention of Table 4 and the second user intention of Table 2. Table 5 is a travel control table showing travel control combining the first user intention and the second user intention. In Table 5, a radius R12 is larger than a radius R11. Moreover, a speed V0 is a minimum speed at which the single-seat electric vehicle 1a can travel. The multi-input controller 102 determines travel control corresponding to the first user intention and the second user intention based on the travel control table of Table 5. Furthermore, the multi-input controller 102 generates a control signal for controlling the motive power source 18 based on the travel control. The motive power source 18 operates in accordance with the control signal. Thereby, the single-seat electric vehicle 1a can make traveling of moving forward, moving backward, turning left, and turning right.

### [Table 5]

**TABLE 5**

| | | | Second user intention | | | | |
|---|---|---|---|---|---|---|---|
| | | | Right turn with a small radius | Right turn with a large radius | No turn | Left turn with a large radius | Left turn with a small radius |
| First user intention | Moving forward | Moving forward & acceleration | Right turn with a radius R11 while acceleration | Right turn with a radius R12 while acceleration | Moving forward while acceleration | Left turn with a radius R12 while acceleration | Left turn with a radius R11 while acceleration |
| | | Maintaining speed | Right turn with a radius R11 without acceleration/deceleration | Right turn with a radius R12 without acceleration/ deceleration | Moving forward without acceleration/deceleration | Left turn with a radius R12 without acceleration/deceleration | Left turn with a radius R11 without acceleration/deceleration |
| | | Moving forward & deceleration | Right turn with a radius R11 while deceleration | Right turn with a radius R12 while deceleration | Moving forward while deceleration | Left turn with a radius R12 while deceleration | Left turn with a radius R11 while deceleration |
| | Stopping | Start moving forward | Right turn with a radius R11 while moving forward at a speed VO | Right turn with a radius R12 while moving forward at a speed V0 | Moving forward at a speed V0 | Left turn with a radius R12 while moving forward at a speed V0 | Left turn with a radius R11 while moving forward at a speed V0 |
| | | Stopping | Right turn on the spot | Right turn on the spot | Stopping | Left turn on the spot | Left turn on the spot |
| | | Start moving backward | Right turn with a radius R11 while moving backward at a speed V0 | Right turn with a radius R12 while moving backward at a speed V0 | Moving backward at a speed V0 | Left turn with a radius R12 while moving backward at a speed V0 | Left turn with a radius R11 while moving backward at a speed V0 |
| | Moving backward | Moving backward & deceleration | Right turn with a radius R11 while deceleration | Right turn with a radius R12 while deceleration | Moving backward while deceleration | Left turn with a radius R12 while deceleration | Left turn with a radius R11 while deceleration |
| | | Maintaining speed | Right turn with a radius R11 without acceleration/deceleration | Right turn with a radius R12 without acceleration/deceleration | Moving backward without acceleration/deceleration | Left turn with a radius R12 without acceleration/deceleration | Left turn with a radius R11 without acceleration/decel eration |
| | | Moving backward & acceleration | Right turn with a radius R11 while acceleration | Right turn with a radius R12 while acceleration | Moving backward while acceleration | Left turn with a radius R12 while acceleration | Left turn with a radius R11 while acceleration |

### [Action of multi-input controller]

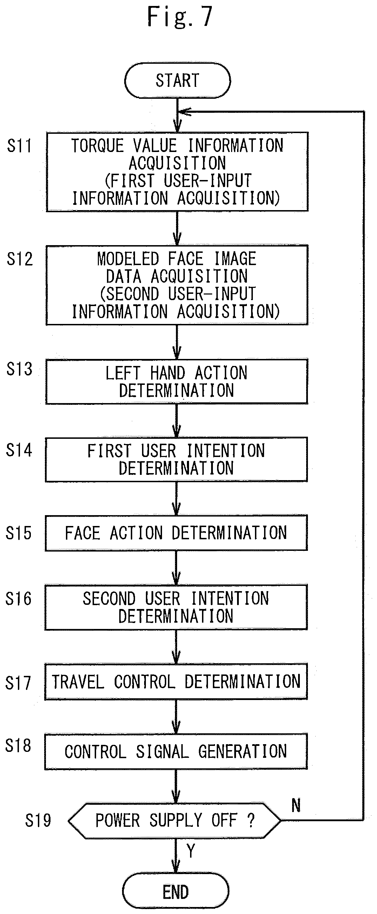

Next, the action of the multi-input controller 102 will be described with reference to the drawings. FIG. 7 is a flowchart showing the action of the multi-input controller 102. The multi-input controller 102 executes the flowchart of FIG. 7 in accordance with the program stored in the storage unit 108.

The present control is started by switching the power supply of the single-seat electric vehicle 1a from OFF to ON. The torque sensor 204a detects a torque value, and outputs torque value information Tr to the user-input information acquisition unit 103. The user-input information acquisition unit 103 acquires torque value information Tr (step S11). The user-input information acquisition unit 103 outputs torque value information to the multi-input controller 102. Thereby, the multi-input controller 102 acquires the torque value information Tr.

In addition, the image sensor 106a picks up an image of the face of the user 200, and outputs face image data FID to the analysis unit 106b. The analysis unit 106b generates modeled face image data MFID based on the face image data FID, and outputs the modeled face image data MFID to the user-input information acquisition unit 105. The user-input information acquisition unit 105 acquires the modeled face image data MFID (step S12). The user-input information acquisition unit 103 outputs the modeled face image data FLID to the multi-input controller 102. Thereby, the multi-input controller 102 acquires the modeled face image data FLID.

Next, the multi-input controller 102 determines which one of "rotating the left handrim 20L in the forward rotation direction", "not rotating the left handrim 20L" or "rotating the left handrim 20L in the reverse rotation direction" is the left hand action indicated by the torque value information Tr (step S13). Furthermore, based on the first user-intention table of Table 4, the multi-input controller 102 determines which one of "accelerating forward movement of the single-seat electric vehicle 1a", "maintaining the speed of forward movement of the single-seat electric vehicle 1a", "decelerating forward movement of the single-seat electric vehicle 1a", "starting forward movement of the single-seat electric vehicle 1a", "stopping the single-seat electric vehicle 1a", "starting backward movement of the single-seat electric vehicle 1a", "decelerating backward movement of the single-seat electric vehicle 1a", "maintaining the speed of backward movement of the single-seat electric vehicle 1a", or "accelerating backward movement of the single-seat electric vehicle 1a" is the first user intention corresponding to the left hand action determined in step S13 (step S14).

Next, the multi-input controller 102 determines which one of "tilting the neck largely in the leftward direction L", "tilting the neck slightly in the leftward direction L", "not tilting the neck", "tilting the neck slightly in the rightward direction R" or "tilting the neck largely in the rightward direction R" of FIG. 4 is the face action indicated by the modeled face image data MFID (step S15). Furthermore, based on the second user-intention table of Table 2, the multi-input controller 102 determines which one of "causing the single-seat electric vehicle 1a to make a left turn with a small radius", "causing the single-seat electric vehicle 1a to make a left turn with a large radius", "causing the single-seat electric vehicle 1a to make neither a left turn nor a right turn", "causing the single-seat electric vehicle 1a to make a right turn with a large radius" or "causing the single-seat electric vehicle 1a to make a right turn with a small radius" is the second user intention corresponding to the face action determined in step S15 (step S16).

Next, the multi-input controller 102 determines travel control corresponding to the first user intention and the second user intention, which are determined in steps S14 and S16 based on the travel control table of Table 5 (step S17). The multi-input controller 102 generates a control signal for controlling the motive power source 18 based on the travel control determined in step S17 (step S18). The motive power source 18 operates in accordance with the control signal.

Finally, the multi-input controller 102 determines whether or not the power supply of the single-seat electric vehicle 1a has been switched from ON to OFF (step S19). When the power supply of the single-seat electric vehicle 1a has been switched from ON to OFF, the present process ends. When the power supply of the single-seat electric vehicle 1a has not been switched from ON to OFF, the present process returns to step S11.

### [Effects]

In the single-seat electric-vehicle travel control apparatus 101a, the user-input detection device 204 detects a torque value not by the image sensor but by the left handrim 20L and the torque sensor 204a, with which the left hand of the user 200 comes into contact. Thus, if the user-input detection device 106 detects a face action by the image sensor 106a, the user-input detection device 204 may not include the image sensor. Even with the single-seat electric-vehicle travel control apparatus 101a as described above, it is possible to suppress the decrease in diversity of travel control in the single-seat electric vehicle 1a for the same reason as with the single-seat electric-vehicle travel control apparatus 101. Further, according to the single-seat electric-vehicle travel control apparatus 101a, the versatility of the user-input detection device 106 is improved for the same reason as with the single-seat electric-vehicle travel control apparatus 101. Further, according to the single-seat electric-vehicle travel control apparatus 101a, the multi-input controller 102 can easily determine the second user intention for the same reason as with the single-seat electric-vehicle travel control apparatus 101.

### (Third embodiment)

### [General configuration of single-seat electric vehicle]

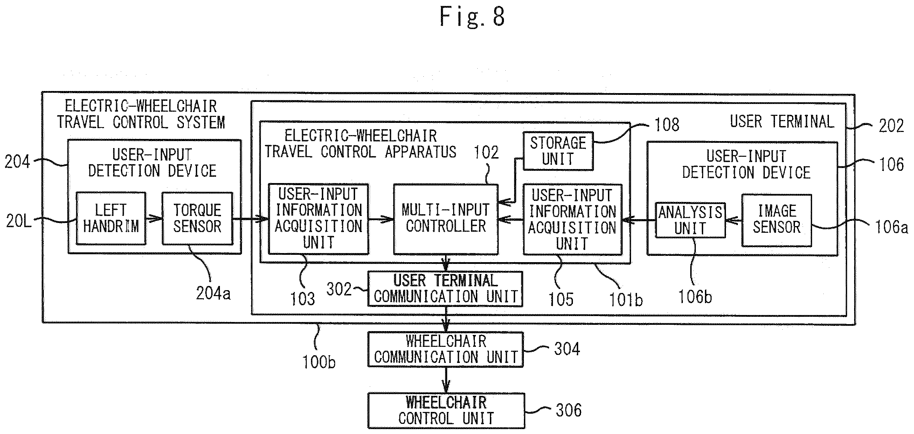

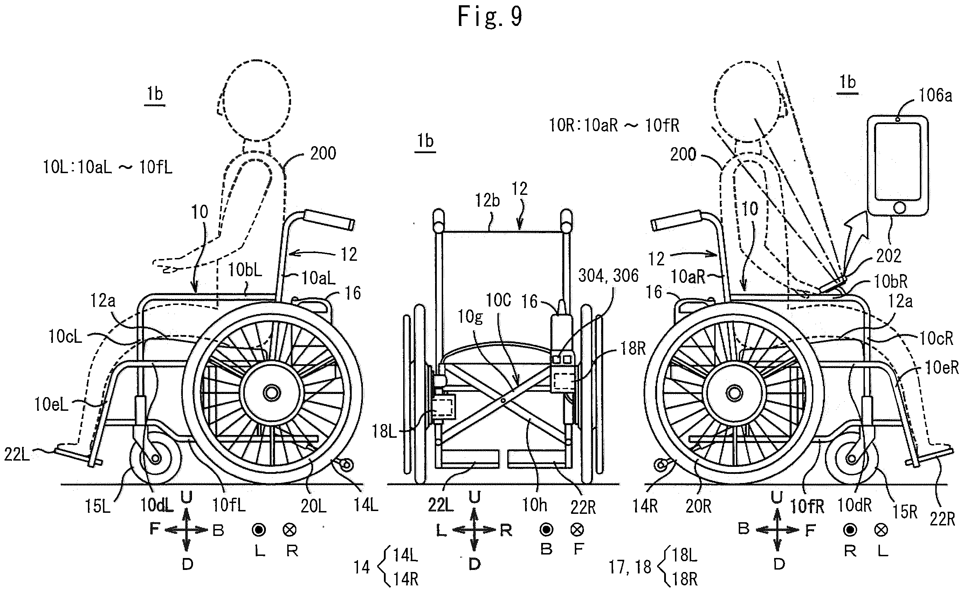

Hereinafter, the general configuration of a single-seat electric vehicle 1b will be described with reference to the drawings. FIG. 8 is a block diagram of a single-seat electric-vehicle travel control system 100b. FIG. 9 shows a view of the single-seat electric vehicle 1b viewed from behind B, a view of the single-seat electric vehicle 1b viewed from the left L, and a view of the single-seat electric vehicle 1b viewed from the right R.

The single-seat electric vehicle 1b according to the present embodiment is an electric wheelchair. The single-seat electric-vehicle travel control system 100b differs from the single-seat electric-vehicle travel control system 100a in the hardware configuration. In the single-seat electric-vehicle travel control system 100a, as shown in FIG. 6, the single-seat electric-vehicle travel control apparatus 101a is a dedicated apparatus for controlling the single-seat electric vehicle 1a. On the other hand, in the single-seat electric-vehicle travel control system 100b, the single-seat electric-vehicle travel control apparatus 101b is a part of a user terminal 202. Therefore, the single-seat electric-vehicle travel control apparatus 101b is not a dedicated apparatus for controlling the single-seat electric vehicle 1b.

The single-seat electric-vehicle travel control system 100b includes a user-input detection device 104 and a user terminal 202. Since the user-input detection device 104 of the single-seat electric-vehicle travel control system 100b is the same as the user-input detection device 104 of the single-seat electric-vehicle travel control system 100a, the description thereof will be omitted.

The user terminal 202 is a wireless communication terminal carried by the user. The user terminal 202 can communicate with other terminals via a telephone line or the Internet. The user terminal 202 can transmit character data, image data, voice data or moving image data to another user terminal, and can receive character data, image data, voice data or moving image data from another user terminal. Moreover, the user terminal 202 can download a web page from a server and can display the web page on a display unit (not shown) of the user terminal 202 or can download software from the server. Further, the user of the user terminal 202 can use the user terminal 202 to talk with the users of other user terminals. Such a user terminal 202 is, for example, a smartphone as shown in FIG. 9.

As shown in FIG. 8, the user terminal 202 includes a single-seat electric-vehicle travel control apparatus 101b, a user-input detection device 106, and a user terminal communication unit 302. Since the single-seat electric-vehicle travel control apparatus 101b of the single-seat electric-vehicle travel control system 100b is the same as the single-seat electric-vehicle travel control apparatus 101a of the single-seat electric-vehicle travel control system 100a, description thereof will be omitted. The single-seat electric-vehicle travel control apparatus 101b is, for example, an SoC (System on a Chip) of a smartphone.

Since the user-input detection device 106 of the single-seat electric-vehicle travel control system 100b is the same as the user-input detection device 106 of the single-seat electric-vehicle travel control system 100a, the description thereof will be omitted. The image sensor 106a is, for example, a camera of a smartphone. Note that the analysis unit 106b may be, for example, a part of the SoC of a smartphone.

The user terminal communication unit 302 performs wireless communication with a wheelchair communication unit 304 (to be described later). The communication standard between the user terminal communication unit 302 and the wheelchair communication unit 304 is, for example, BlueTooth (registered trademark). However, the user terminal communication unit 302 and the wheelchair communication unit 304 may be connected by wire.

As shown in FIG. 9, the single-seat electric vehicle 1b includes a wheelchair communication unit 304 and a wheelchair control unit 306. The wheelchair communication unit 304 wirelessly communicates with the user terminal communication unit 302. The wheelchair control unit 306 performs travel control of the single-seat electric vehicle 1b. Specifically, the wheelchair control unit 306 acquires a control signal generated by the multi-input controller 102 via the user terminal communication unit 302 and the wheelchair communication unit 304. Furthermore, the wheelchair control unit 306 controls the action of the motive power source 18 based on the acquired control signal.

Since the action of the single-seat electric-vehicle travel control system 100b is the same as the action of the single-seat electric-vehicle travel control system 100a, description thereof will be omitted.

### [Effects]

Even with the single-seat electric-vehicle travel control apparatus 101b as described above, it is possible to suppress decrease in diversity of travel control in the single-seat electric vehicle 1b for the same reason as with the single-seat electric-vehicle travel control apparatus 101a. Further, according to the single-seat electric-vehicle travel control apparatus 101b, the versatility of the user-input detection device 106 is improved for the same reason as with the single-seat electric-vehicle travel control apparatus 101a. Further, according to the single-seat electric-vehicle travel control apparatus 101b, the multi-input controller 102 can easily determine the second user intention for the same reason as with the single-seat electric-vehicle travel control apparatus 101a.

### (Other embodiments)

The embodiments and variations which at least have been either described or illustrated herein are for the purpose of facilitating the understanding of the present disclosure, and are not intended to limit the spirit of the present disclosure. The above embodiments and variations can be modified and improved without departing from the scope of the teaching.

The spirit includes equivalent elements, modifications, deletions, combinations (for example, combinations of features across embodiments and variations), improvements, and alterations that can be recognized by those skilled in the art based on the exemplary embodiments disclosed herein. The limitations in the claims should be interpreted broadly based on the terms used in the claims, and should not be limited to the embodiments and variations set forth herein or in the prosecution of the present application. Such embodiments and variations should be construed as non-exclusive. For example, in the present specification, the terms "preferably" and "good" are non-exclusive and mean "preferable but not limited thereto", "good but not limited thereto".

Note that in the single-seat electric-vehicle travel control apparatus 101, the user-input detection devices 104 and 106 detect a left hand action and a face action, respectively. However, the user-input detection devices 104 and 106 may respectively detect action of any part of the body of the user 200 other than the left hand and the face. The part of the body of the user 200 other than the left hand and the face includes, for example, the head, jaw, eyeballs, eyelids, nose, mouth, tongue, ears, right hand, shoulders, arms, elbows, knees, feet, and the like. Also, the user-input detection devices 104 and 106 may respectively detect actions of a first user-input representing member and a second user-input representing member, which are supported by the body of the user 200, instead of the action of a part of the body of the user 200. Examples of the first user-input representing member and the second user-input representing member include a rod-like member held in the hand by the user 200 or a rod-like member held in the mouth by the user 200.

Further, in the single-seat electric-vehicle travel control apparatuses 101a and 101b, the user-input detection device 106 detects a face action. However, the user-input detection device 106 may detect action of any part of the body of the user 200 other than the face. The part of the body of the user 200 other than the face includes, for example, the head, jaw, eyeballs, eyelids, nose, mouth, tongue, ears, hands, shoulders, arms, elbows, knees, feet, and the like. The user-input detection device 106 may detect an action of the second user-input representing member supported by the body of the user 200, instead of an action of a part of the body of the user 200, as the second user input by the image sensor 106a. Examples of the second user-input representing member include a rod-like member held in the hand by the user 200 or a rod-like member held in the mouth by the user 200.

Further, in the single-seat electric-vehicle travel control apparatuses 101a and 101b, the user-input detection device 204 comes into contact with the left hand of the user 200 to detect the left hand action. However, in the single-seat electric-vehicle travel control apparatuses 101a and 101b, the user-input detection device 204 may detect the action of the first user-input representing member supported by the body of the user 200 by coming into contact with the first user-input representing member.

Further, in the single-seat electric-vehicle travel control apparatuses 101a and 101b, the user-input detection device 204 detects the left hand action by the left handrim 20L and the torque sensor 204a. However, the user-input detection device 204 may detect action of a part of the body of the user 200 other than the left hand. The part of the body of the user 200 other than the left hand includes, for example, at least one of the head, jaw, face, eyeballs, eyelids, nose, mouth, tongue, ears, right hand, shoulders, arms, elbows, knees, feet and the like. Thus, the part of the body of the user 200 other than the left hand may be combinations of two or more parts of the head, jaw, face, eyeballs, eyelids, nose, mouth, tongue, ears, right hand, shoulders, arms, elbows, knees, feet, and the center of gravity of the body. Moreover, the action of the eyeball includes the movement of the line of sight and the movement of the iris of the eye.

In the single-seat electric-vehicle travel control apparatuses 101a and 101b, the user-input detection device 204 includes a left handrim 20L and a torque sensor 204a to detect the left hand action. However, the user-input detection device 204 may detect the left hand action with any configuration other than that of the left handrim 20L and the torque sensor 204a. The user-input detection device 204 may include, instead of the left handrim 20L and the torque sensor 204a, for example, a joystick, a handle, a lever, a button or the like, with which a part of the user's body comes into contact. The joystick, the handle, the lever or the button may be, for example, an input apparatus for steering for controlling left turn and right turn of the single-seat electric vehicle 1a, 1b, or an input apparatus for an accelerator or a brake for controlling the forward movement and backward movement of the single-seat electric vehicle 1a, 1b.

Note that the single-seat electric vehicle is not limited to an electric wheelchair. Specifically, in the single-seat electric vehicles 1, 1a and 1b, the motive power source 18 causes a difference in the rotational speed between the left drive wheel 14L and the right drive wheel 14R when causing the single-seat electric vehicle to make a left turn or a right turn. However, the single-seat electric vehicle may be, for example, a vehicle provided with one or two steerable wheels and one or two drive wheels. One or two steerable wheels are operated by a handle, a joystick or the like. The two drive wheels are rotated by the motive power source 18. The motive power source 18 is operated by an accelerator pedal or an accelerator lever. In this case, the traveling means 17 includes a steering mechanism, which is provided between a handle or a joystick and one or two steerable wheels, and the motive power source 18. The user-input detection device 104 also includes a handle and a joystick, etc., and an accelerator pedal or an accelerator lever. The user-input detection device 106 includes an image sensor 106a. The image sensor 106a picks up, for example, an image of the whole of an upper half body or a part of the upper half body of the user 200. The multi-input controller 102 determines that the second user intention is stopping the single-seat electric vehicle upon acquisition of second user-input information indicative of a second user action in which a state of the upper half body of the user 200 extending in the upward direction U from the seat 12 changes into a state of the upper half body of the user 200 being fallen in the frontward direction F. The multi-input controller 102 determines that the second user intention is stopping the single-seat electric vehicle upon acquisition of second user-input information indicative of the second user action meaning a poor physical condition of the user 200. The second user action that means a poor physical condition of the user 200 includes, in addition to the upper half body of the user falling in the frontward direction F, the user 200 leaning on a backrest 12b of the seat 12 with the face of user 200 facing upward, and the user 200 falling in the leftward direction L or the rightward direction R. As so far described, in the single-seat electric vehicle, when the upper half body of the user falls in the frontward direction F due to a poor physical condition or the like, the single-seat electric vehicle may be stopped. Note that the traveling means 17 may further include a braking unit that generates a braking force for preventing the rotation of the steerable wheel and/or the drive wheel. Then, the multi-input controller 102 may cause the single-seat electric vehicle to be stopped by the braking unit upon acquisition of second user-input information indicative of a second user action in which a state of the upper half body of the user 200 extending in the upward direction U from the seat 12 changes into a state of the upper half body of the user 200 falling in the frontward direction F.

The single-seat electric vehicle is an electric vehicle with a single passenger capacity. In addition, a single-seat electric vehicle travels at low speed. The speed of an electric wheelchair is set at not more than 6 km/hour according to Japanese regulations. Therefore, the low speed is, for example, not more than 6 km/hour. However, the speed of the electric wheelchair may be more than 6 km/hour in foreign regulations. Therefore, the low speed may be, for example, not more than 10 km/hour. Therefore, the single-seat electric vehicle does not include an EV (Electric Vehicle) that can carry a plurality of people. Moreover, the single-seat electric vehicle does not include, for example, an electric vehicle that can travel at high speed of not less than 100 km/hour on a public road.

Further, in the single-seat electric-vehicle travel control apparatuses 101a and 101b, the user-input detection device 204 may detect the action of the eyelid or the mouth, etc., by an electromyograph attached to the eyelid or the mouth, etc. Moreover, the user-input detection device 204 may detect an inclination of a part of the user's body by an acceleration sensor or a potentiometer attached to the part of the body such as the face or the head of the user 200.

In the single-seat electric-vehicle travel control apparatus 101, the analysis units 104b and 106b may not extract feature points of the left hand and the face, respectively. That is, the multi-input controller 102 may determine the left hand action and the face action based on the left-hand image data LID and the face image data FID. Similarly, in the single-seat electric-vehicle travel control apparatuses 101a and 101b, the analysis unit 106b may not extract feature points of the face. That is, the multi-input controller 102 may determine the face action based on the face image data FID.

Further, the left hand action of FIG. 3 in the single-seat electric-vehicle travel control apparatus 101 is an example, and is not limited to the illustrated left hand action. The left hand action may be, for example, an action of waving the hand up and down or waving the hand left and right. Furthermore, the face action of FIG. 4 in the single-seat electric-vehicle travel control apparatuses 101, 101a, and 101b is an example, and is not limited to the illustrated face action. The face action may be, for example, an action of tilting the face back and forth.

Further, the first user-intention table of Table 1 in the single-seat electric-vehicle travel control apparatus 101 is exemplary and not limited to the aforementioned first user-intention table. For example, the left hand action of "making a fist" may correspond to the first user intention of "stopping the single-seat electric vehicle 1". Also, there may be more types of left hand actions and first user intentions than the left hand actions and the first user intentions of Table 1. This allows to increase the variety of travel control in the single-seat electric vehicle 1.

The second user-intention table of Table 2 in the single-seat electric-vehicle travel control apparatuses 101, 101a, and 101b is exemplary and not limited to the aforementioned second user-intention table. Also, there may be more types of face actions and second user intentions than the face actions and second user intentions of Table 2. This allows to increase the diversity of travel control in single-seat electric vehicles 1, 1a, and 1b.

The single-seat electric-vehicle travel control apparatuses 101, 101a, and 101b perform travel control regarding forward movement and backward movement of the single-seat electric vehicles 1, 1a, and 1b by left hand action, and performs travel control regarding left turn and right turn of the single-seat electric vehicles 1, 1a, and 1b by face action. However, the travel control of the single-seat electric-vehicle travel control apparatuses 101, 101a, and 101b will not be limited to this. The single-seat electric-vehicle travel control apparatuses 101, 101a, and 101b may perform, for example, travel control regarding forward movement and backward movement of the single-seat electric vehicles 1, 1a, and 1b by face action, and perform travel control regarding left turn and right turn of the single-seat electric vehicles 1, 1a and 1b by left hand action.

Moreover, the block diagram of FIG. 1 is a functional block diagram showing the function of each component of the single-seat electric-vehicle travel control apparatus 101. Therefore, the hardware configuration of the single-seat electric-vehicle travel control apparatus 101 may not be in accordance with the block diagram of FIG. 1. For example, the microcomputer of the analysis unit 104b and the microcomputer of the multi-input controller 102 may be constituted by one microcomputer or by two microcomputers. Similarly, the block diagrams of FIG. 6 and FIG. 8 are functional block diagrams showing functions of each component of single-seat electric-vehicle travel control apparatuses 101a and 101b. Therefore, the hardware configuration of the single-seat electric vehicle travel control apparatuses 101a and 101b may not be in accordance with the block diagrams of FIGS. 6 and 8.

The single-seat electric vehicles 1, 1a and 1b shown in FIGS. 1, 6 and 9 are electric wheelchairs of a type in which the electric unit is retrofitted to the wheelchair. However, the single-seat electric vehicles 1, 1a and 1b may be electric wheelchairs of a type which is designed as an electric wheelchair, without being limited to electric wheelchairs of the type in which the electric unit is retrofitted to the wheelchair.

Moreover, in the single-seat electric-vehicle travel control apparatuses 101a and 101b, the multi-input controller 102 may determine the first user intention based on the torque value information Tr without executing step S13. That is, the multi-input controller 102 may determine the first user intention based on the torque value information Tr without determining the left hand action.

Moreover, in the single-seat electric vehicles 1, 1a and 1b, the left drive wheel 14L and the right drive wheel 14R are drive wheels. However, the left drive wheel 14L and the right drive wheel 14R may not be a drive wheel which receives supply of motive power from the motive power source 18. In this case, single-seat electric vehicles 1, 1a and 1b further include a left drive wheel (not shown) and a right drive wheel (not shown), which are provided separately from the left drive wheel 14L and the right drive wheel 14R, and receive supply of motive power from the motive power source 18.

Note that the single-seat electric-vehicle travel control apparatus 101 may include, in addition to the user-input detection devices 104 and 106, a user-input detection device which comes into contact with a part of the user's body to detect the user action and/or a user-input detection device which detects the user action without contacting a part of the user's body. Moreover, the single-seat electric-vehicle travel control apparatuses 101a and 101b may include, in addition to the user-input detection device 204 and the user-input detection device 106, a user-input detection device which comes into contact with a part of the user's body to detect the user action and/or a user-input detection device which detects the user action without contacting a part of the user's body.

Note that, in the single-seat electric-vehicle travel control apparatus 101, the user-input detection devices 104 and 106 respectively detect the left hand action or face action without contacting the left hand or the face of the user 200. Therefore, the user-input detection devices 104 and 106 respectively include image sensors 104a and 106a. However, the user-input detection devices 104 and 106 need only to be able to detect the left hand action or the face action without contacting the left hand or the face of the user 200, respectively. Thus, the user-input detection devices 104, 106 may not include the image sensors 104a and 106a, respectively. In this case, the user-input detection devices 104 and 106 may respectively include a device for detecting the left hand action or the face action without contacting the left hand or the face of the user 200. Such devices include, for example, distance sensors using ultrasonic waves, heat sensors, and the like.

Note that, in the single-seat electric-vehicle travel control apparatuses 101, 101a, and 101b, the multi-input controller 102 determines the face action based on the magnitude of the inclination angle θ formed by the center line CL and the vertical axis Ax. However, the method of determining the face action is not limited to this. For example, the multi-input controller 102 stores, as an initial position, the coordinates of the nose when the face action is "not tilting the neck". Then, the multi-input controller 102 may determine the face action by calculating the amount of displacement of the coordinates of the nose from the initial position.

Note that, in the single-seat electric-vehicle travel control systems 100, 100a, and 100b, the user-input detection devices 104 and 106 are not a part of the single-seat electric-vehicle travel control apparatuses 101, 101a, and 101b. This means both that the user-input detection devices 104, 106, and 204 each have a structure physically independent of the single-seat electric-vehicle travel control apparatuses 101, 101a, and 101b, and that the user-input detection devices 104, 106, and 204 each have a function physically independent of the single-seat electric-vehicle travel control apparatuses 101, 101a, and 101b. The fact that two configurations have physically independent structures means that the two configurations can be physically separated into two. Also, the fact that two configurations have functionally independent structures means that the two configurations can be functionally divided into two. In this case, the two configurations may not be physically separated into two. An example in which the two configurations have functionally independent structures includes, for example, a case in which the analysis unit 104b of the user-input detection device 104 and the single-seat electric-vehicle travel control apparatus 101 are constituted by one microcomputer.

The single-seat electric-vehicle travel control systems 100, 100a, and 100b may further include a display apparatus for showing the magnitude of inclination of the neck. The display apparatus is, for example, five LEDs (Light Emitting Diodes) aligned in a row in the left-right direction. When the face action is "tilting the neck largely in the leftward direction L", the first, second, and third LEDs from the left end light up. When the face action is "tilting the neck slightly in the leftward direction L", the second and third LEDs from the left end light up. When the face action is "not tilting the neck", the third LED from the left en.d lights up. When the face action is "tilting the neck slightly in the rightward direction R", the second and third LEDs from the right end light up. When the face action is "tilting the neck largely in the rightward direction R", the first, second, and third LEDs from the right end light up. This enables the user 200 to recognize the moving direction of the single-seat electric vehicle 1, 1a, 1b.

### REFERENCE SIGNS LIST

- 1, 1a, 1b:: Single-seat electric vehicle
- 10:: Body frame
- 10C:: Body frame connecting part
- 10L:: Body frame left part
- 10R:: Body frame right part
- 10aL:: Left handle frame
- 10aR:: Right handle frame
- 10bL:: Left elbow-rest frame
- 10bR:: Right elbow-rest frame
- 10cL:: Left caster frame
- 10cR:: Right caster frame
- 10dL:: Left seat frame
- 10dR:: Right seat frame
- 10eL:: Left footrest frame
- 10eR:: Right footrest frame
- 10fL:: Left under frame
- 10fR:: Right under frame
- 10g, 10h:: Connecting frame
- 12:: Seat
- 12a:: Seat surface
- 12b:: Backrest
- 14L:: Left drive wheel
- 14R:: Right drive wheel
- 15L:: Left caster
- 15R:: Right caster
- 16:: Power supply
- 17:: Traveling means
- 18:: Motive power source
- 18L:: Left motive power source
- 18R:: Right motive power source
- 20L:: Left handrim
- 20R:: Right handrim
- 22L:: Left footrest
- 22R:: Right footrest
- 100, 100a, 100b:: Single-seat electric-vehicle travel control system
- 101, 101a, 101b:: Single-seat electric-vehicle travel control apparatus
- 102:: Multi-input controller
- 104, 106:: User-input detection device
- 104a, 106a:: Image sensor
- 104b, 106b:: Analysis unit
- 108:: Storage unit
- 200:: User
- 202:: User terminal
- 204:: User-input detection device
- 204a:: Torque sensor
- 302:: User terminal communication unit
- 304:: Wheelchair communication unit
- 306:: Wheelchair control unit
- FID:: Face image data
- FLID:: Modeled face image data
- LID:: Left-hand image data
- MFID:: Modeled face image data
- MLID:: Modeled left-hand image data

## Claims

1. A single-seat electric-vehicle travel control apparatus for performing travel control of a single-seat electric vehicle, the single-seat electric vehicle comprising:
a body frame;
a seat supported by the body frame, the seat being for a user to sit;
one or more drive wheels supported by the body frame;
a power supply supported by the body frame; and
traveling means including a motive power source rotating the one or more drive wheels by receiving supply of power from the power supply, the traveling means being capable of causing the single-seat electric vehicle to move forward and backward, and to make a left turn and a right turn, wherein
a first user-input detection device is not a part of the single-seat electric-vehicle travel control apparatus, and detects a first user action of a first part of the user's body or a first user action of a first user-input representing member supported by the user's body to output first user-input information indicative of the first user action,
a second user-input detection device is not a part of the single-seat electric-vehicle travel control apparatus, and detects a second user action of a second part of the user's body or a second user action of a second user-input representing member supported by the user's body without contacting the second part of the user's body or the second user-input representing member to output second user-input information indicative of the second user action;
the second part of the user's body is a part different from the first part of the user's body; and
the second user-input representing member is a member different from the first user-input representing member, and
the single-seat electric-vehicle travel control apparatus comprising:
a first user-input information acquisition unit of (A);
a second user-input information acquisition unit of (B); and
a multi-input controller of (C), wherein
(A): the first user-input information acquisition unit acquires the first user-input information indicative of the first user action detected by the first user-input detection device,
(B): the second user-input information acquisition unit acquires the second user-input information indicative of the second user action detected by the second user-input detection device without contact with the second part of the user's body or the second user-input representing member,
(C): the multi-input controller determines a first user intention regarding travel control of the single-seat electric vehicle based on the first user-input information acquired by the first user-input information acquisition unit,
the multi-input controller determines a second user intention regarding travel control of the single-seat electric vehicle based on the second user-input information indicative of the second user action detected by the second user-input detection device without contact with the second part of the user's body or the second user-input representing member, and
the multi-input controller generates a control signal for controlling the traveling means based on the first user intention and the second user intention.

2. The single-seat electric-vehicle travel control apparatus according to claim 1, wherein
the one or more drive wheels include a left drive wheel supported by the body frame at a position further leftward than a center of the body frame in a body frame left-right direction, and a right drive wheel supported by the body frame at a position further rightward than the center of the body frame in the body frame left-right direction, and
the motive power source causes a difference between a rotational speed of the left drive wheel and a rotational speed of the right drive wheel when causing the single-seat electric vehicle to make a left turn or a right turn.

3. A single-seat electric-vehicle travel control system, comprising:
the first user-input detection device;
the second user-input detection device; and
the single-seat electric-vehicle travel control apparatus according to claim 1 or 2.

4. A single-seat electric-vehicle travel control system according to claim 3, wherein
the second user-input detection device includes an image sensor.

5. A single-seat electric-vehicle travel control system according to claim 3 or 4, wherein
the first user-input detection device detects the first user action of the first part of the user's body or the first user action of the first user-input representing member without contacting the first part of the user's body or the first user-input representing member.

6. A single-seat electric-vehicle travel control system according to claim 5, wherein
the first user-input detection device includes an image sensor.

7. A single-seat electric-vehicle travel control system according to claim 3, wherein
the first user-input detection device detects the first user action of the first part of the user's body or the first user action of the first user-input representing member by contacting the first part of the user's body or the first user-input representing member.

8. A single-seat electric-vehicle travel control system according to claim 7, wherein
the second user-input detection device includes an image sensor, and
the multi-input controller determines that the second user intention is stopping the single-seat electric vehicle upon acquisition of the second user-input information indicative of the second user action which implies poor physical condition of the user.

9. The single-seat electric-vehicle travel control system according to claim 7 or 8, wherein
the first user-input detection device detects the first user action of the first part of the user's body, and
the first user-input detection device includes any one of a joystick, a handle, a lever, a button, or a handrim, with which the first part of the user's body comes into contact.

10. A single-seat electric-vehicle travel control system according to any one of claims 3 to 9, wherein
the second user-input detection device detects at least one of the second user actions of the head, jaw, face, eyeballs, eyelids, nose, mouth, tongue, ears, shoulders, hands, arms, elbows, knees, feet or a center of gravity of the body of the user.

11. A single-seat electric-vehicle travel control system according to any one of claims 3 to 10, wherein
the second user-input detection device detects the second user action by extracting a plurality of feature points in the second part of the user's body.

12. A single-seat electric vehicle, comprising:
the body frame;
a seat supported by the body frame, the seat being for the user to sit;
one or more drive wheels supported by the body frame;
a power supply supported by the body frame;
traveling means including a motive power source for rotating the one or more drive wheels by receiving supply of power from the power supply, the traveling means being capable of causing a single-seat electric vehicle to move forward and backward, and to make a left turn and a right turn; and
the single-seat electric-vehicle travel control system according to any one of claims 3 to 11.
